(19) Europäisches Patentamt
European Patent Office
Office européen des brevets

(11) EP 1 178 846 B1

(12) **EUROPEAN PATENT SPECIFICATION**

(45) Date of publication and mention of the grant of the patent:
**02.09.2009 Bulletin 2009/36**

(21) Application number: **99953011.6**

(22) Date of filing: **01.10.1999**

(51) Int Cl.:
***A61L 15/34*** *(2006.01)* ***A61F 13/15*** *(2006.01)*

(86) International application number:
**PCT/US1999/022842**

(87) International publication number:
**WO 2000/069483 (23.11.2000 Gazette 2000/47)**

(54) **ABSORBENT ARTICLE WITH SKIN CARE COMPOSITION**

SAUGFÄHIGER ARTIKEL MIT HAUTPFLEGEMITTEL

ARTICLE ABSORBANT COMPRENANT UNE COMPOSITION DE SOIN POUR LA PEAU

(84) Designated Contracting States:
**AT BE CH CY DE DK ES FI FR GB GR IE IT LI LU MC NL PT SE**

(30) Priority:
**19.05.1999 PCT/US99/10986**
**21.05.1999 PCT/US99/11330**
**21.05.1999 PCT/US99/11374**
**21.05.1999 PCT/US99/11372**

(43) Date of publication of application:
**13.02.2002 Bulletin 2002/07**

(73) Proprietor: **THE PROCTER & GAMBLE COMPANY**
**Cincinnati, Ohio 45202 (US)**

(72) Inventors:
- **GRAY, Brian Francis**
  **Kobe 658-0032 (JP)**
- **MINOGUCHI, Ryo**
  **Higashinada-ku,**
  **Kobe 658-0056 (JP)**

(74) Representative: **Kremer, Véronique Marie Joséphine et al**
**Procter & Gamble Service GmbH**
**Sulzbacher Strasse 40-50**
**65824 Schwalbach am Taunus (DE)**

(56) References cited:
**WO-A-99/12530**
**WO-A-99/12583**
**WO-A-99/22684**
**WO-A-99/25287**
**DE-C- 3 309 530**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### TECHNICAL FIELD

This application relates to feminine hygiene absorbent articles, such as sanitary napkins and panti-liners. More particularly, the present invention relates to absorbent articles having a skin care composition coating on the body contacting surface of the absorbent article that is transferable to the wearer's skin.

### BAGKGROUND OF THE INVENTION

All manner and variety of absorbent articles configured for the absorption of body fluids such as menses, urine and feces are, of course, well known. With respect to feminine protection devices, the art has offered two basic types; sanitary napkins have been developed for external wear about the pudendal region while tampons have been developed for internal wear within the vaginal cavity for interruption of menstrual flow therefrom. Such tampon devices are disclosed in U.S. Patent No. 4,412,833, entitled "Tampon Applicator", issued to Weigner, et al. on November 1, 1983, and U.S. Patent No. 4,413,986, entitled "Tampon Assembly With Means For Sterile Insertion", issued to Jacobs on November 8, 1983.

Hybrid devices which attempt to merge the structural features of the sanitary napkins and the tampons into a single device have also been proposed. Such hybrid devices are disclosed in U.S. Patent No. 2,092,346, entitled "Catamenial Pad", issued to Arone on September 7, 1937, and U.S. Patent No. 3,905,372, entitled "Feminine Hygiene Protective Shield", issued to Denkinger on September 16, 1975. Other less intrusive hybrid devices are known as labial or interlabial sanitary napkins and are characterized by having a portion which at least partially resides within the wearer's vestibule and a portion which at least partially resides external of the wearer's vestibule. Such devices are disclosed in U.S. Patent No. 2,662,527, entitled "Sanitary Pad", issued to Jacks on December 15, 1953, and U.S. Patent No. 4,631,062, entitled "Labial Sanitary Pad", issued to Lassen, et al. on December 23, 1986. Interlabial pads have the potential to provide even greater freedom from inconvenience because of their small size and reduced risk of leakage. Numerous attempts have been made in the past to produce an interlabial pad which would combine the best features of tampons and sanitary napkins while avoiding at least some of the disadvantages associated with each of these types of devices. Examples of such devices are described in U.S. Patent 2,917,049 issued to Delaney on December 15, 1959, U.S. Patent 3,420,235 issued to Harmon on January 7, 1969, U.S. Patent 4,595,392 issued to Johnson, et al. on June 17, 1986, and U.S. Patent 5,484,429 issued to Vukos, et al. on January 16, 1996. A commercially available interlabial device is FRESH 'N FIT® PADETTE interlabial product which is marketed by Athena Medical Corp. of Portland, OR and described in U.S. Patents 3,983,873 and 4,175,561 issued to Hirschman on October 5, 1976 and November 27, 1979, respectively.

Although these products such as sanitary napkins, tampons and interlabial pads are effective generally to absorb menses, there are still discomfort which need to be solved. For example, the wearer feels uncomfortable due to; (1) frictional discomfort associated with rubbing of the product against the wearer's skin while wearing/applying the product; (2) adherence of the menses discharged onto the topsheet to the wearer's skin; and (3) adherence of the surface of the product such as a topsheet to the wearer's skin. The friction when applying the product prevents the product from being properly inserted/applied, leading to discomfort. In addition, rubbing of the product against the wearer's skin causes itch and/or skin irritation. The adherence of the menses gives the wearer messy/dirty feeling. In addition, enzyme and/or microbial contained in the adhered menses attack the wearer's skin, thereby causing itch and/or skin irritation. The adherence of the topsheet gives the wearer sticky feeling. It also hinders the wearer's skin from air circulation, thereby causing skin overhydration.

Thus, it would be desirable to apply skin care compositions on absorbent articles, which deliver skin care benefits, e.g., to reduce wearer's discomfort or to address the concerns of skin disorders associated with wearing absorbent articles. The skin care compositions are applied on the body contacting surface of the absorbent article such that the skin care compositions can transfer from the absorbent article to the wearers body by a contact. The major portion of the body contacting surface is typically a top surface of the absorbent article to absorb the body exudates such as urine or menses therethrough. Therefore, applying too much skin care compositions on the skin contacting surface hinders body exudates from being absorbed into the absorbent core of the absorbent article.

Thus, there is a need for an absorbent article which absorbs body exudates effectively while delivering skin care benefits to the wearer's skin.

### SUMMARY OF THE INVENTION

The present invention relates to a feminine hygiene absorbent article having a skin care composition according to claim 1. The absorbent article has a body surface and a garment surface. The absorbent article comprises a topsheet disposed at the body surface, a backsheet disposed at the garment surface, and an absorbent core disposed therebetween. At least a portion of the absorbent article is provided with a skin care composition. The absorbent article has a preferential acquisition zone and a skin care zone. The preferential acquisition zone covers at least a portion of the vulva of the wearer when the absorbent article is applied on the wearer's body. The skin care zone is provided with the skin care composition of greater basis weight than the preferential acquisition zone.

## BRIEF DESCRIPTION OF THE DRAWINGS

While the specification concludes with claims particularly printing out and distinctly claiming the subject matter which is regarded as forming the present invention, it is believed that the invention will be better understood from the following description which is taken in conjunction with the accompanying drawings in which:

FIG. 1 is an absorbent article in the form of a sanitary napkin according to the present invention;

FIG. 2 is a lateral cross-sectional view taken along line 2-2 of the sanitary napkin shown in FIG. 1;

FIG. 3 is a lateral cross-sectional view taken along line 3-3 of FIG. 1 through the center portion of one of the flaps;

FIG. 4 is a top plan view of the absorbent article showing examples of a preferential acquisition zone and a skin care zone,

FIG. 5 is a top plan view of the absorbent article showing alternative examples of a preferential acquisition zone and skin care zones, and

FIG. 6 is a graph of shear stress vs. viscosity of one example of skin care composition.

## DETAILED DESCRIPTION OF THE INVENTION

"Comprising" means that other steps and other elements which do not affect the end result can be added. This term encompasses the terms "consisting of and "consisting essentially of".

All percentages, ratios and proportions used herein are by weight unless otherwise specified.

### A. Absorbent Article

Herein "absorbent article" refers to devices which absorb and contain body exudates (e.g., body fluid), and more specifically, refers to devices which are placed against the skin of a wearer to absorb and contain the various exudates discharged from the body. Herein "disposable" is used to describe absorbent articles which are not intended to be laundered or otherwise restored or reused as an absorbent article after a single use. Examples of disposable (feminine hygiene) absorbent articles include sanitary napkins and panti-liners.

Disposable absorbent articles typically comprise a liquid pervious topsheet, a liquid impervious backsheet joined to the topsheet and an absorbent core positioned between the topsheet and the backsheet. Disposable absorbent articles and components thereof, including the topsheet, backsheet, absorbent core, and any individual layers of these components, have a body surface and a garment surface. Herein "body surface" means that surface of the article or component which is intended to be worn toward or adjacent to the body of the wearer, while the "garment surface" is on the opposite side and is intended to be worn toward or placed adjacent to the wearer's body or undergarments when the disposable absorbent article is worn.

As used herein, the term "body contacting surface" of an absorbent article is one or more surfaces of any article components that contact the wearer at some time during the wear period. Body contacting surfaces include, but are not limited to, portions of the topsheet, flaps which contact a wearer during use.

Disposable absorbent articles also have a preferential acquisition zone on the body surface and a skin care zone on the body contacting surface. Herein, the term "preferential acquisition zone" means the zone on the body surface which preferentially acquires body exudatess. The preferential acquisition zone may be provided with a skin , care composition to deliver skin care benefits to the wearer. Herein, the term "skin care zone" means the zone on the body contacting surface which delivers skin care benefits to the wearer through the transfer of the skin care composition applied on the skin care zone. The skin care zone may be able to absorb body exudatess.

The following description generally discusses the absorbent core, topsheet, and backsheet materials that are useful in disposable absorbent articles, though disposable absorbent article does not necessarily need to have all of them. It is to be understood that this general description applies to these components of the specific absorbent articles shown in FIGS. 1-3 and further described below, in addition to those of other disposable absorbent articles which are

generally described herein.

In general, the absorbent core is capable of absorbing or retaining liquids (e.g., menses, urine, and/or other body exudates). The absorbent core is preferably compressible, conformable, and non-irritating to the wearer's skin. The absorbent core may be manufactured in a wide variety of sizes and shapes (e.g., rectangular, oval, hourglass, "T" shaped, dog bone, asymmetric, etc.). The absorbent core may include any of a wide variety of liquid-absorbent materials commonly used in absorbent articles, such as comminuted wood pulp, which is generally referred to as airfelt. Examples of other suitable absorbent materials for use in the absorbent core include creped cellulose wadding; meltblown polymers including coform; chemically stiffened, modified or cross-linked cellulosic fibers; synthetic fibers such as crimped polyester fibers; peat moss; tissue including tissue wraps and tissue laminates; absorbent foams; absorbent sponges; superabsorbent polymers; absorbent gelling materials; or any equivalent material or combinations of materials, or mixtures of these.

The configuration and construction of the absorbent core may also be varied (e.g., the absorbent core may have varying caliper zones and/or have a profile so as to be thicker in the center; hydrophilic gradients; gradients of the absorbent composite, superabsorbent gradients; or lower average density and lower average basis weight zones, e.g., acquisition zones; or may comprise one or more layers or structures). The total absorbent capacity of the absorbent core should, however, be compatible with the design loading and the intended use of the absorbent article. Further, the size and absorbent capacity of the absorbent core may be varied to accommodate different uses such as diapers, incontinence pads, pantiliners, regular sanitary napkins, and overnight sanitary napkins, and to accommodate wearers ranging from infants to adults.

The absorbent core can include other absorbent components that are often used in absorbent articles, for example, a dusting layer, a wicking or acquisition layer, or a secondary topsheet for increasing the wearer's comfort.

The topsheet is preferably compliant, soft feeling, and non-irritating to the wearer's skin. Further, the topsheet is liquid pervious, permitting liquids (e.g., menses and/or urine) to readily penetrate through its thickness. A suitable topsheet may be manufactured from a wide range of materials such as woven and nonwoven materials (e.g., a nonwoven web of fibers); polymeric materials such as apertured formed thermoplastic films, apertured plastic films, and hydro formed thermoplastic films; porous foams; reticulated foams; reticulated thermoplastic films; and thermoplastic scrims. Suitable woven and nonwoven materials can be comprised of natural fibers (e.g., wood or cotton fibers), synthetic fibers (e.g., polymeric fibers such as polyester, polypropylene, or polyethylene fibers) or from a combination of natural and synthetic fibers. When the topsheet comprises a nonwoven web, the web may be manufactured by a wide number of known techniques. For example, the web may be spunbonded, carded, wet-laid, meltblown, hydroentangled, combinations of the above, or the like.

The backsheet is impervious to liquids (e.g., menses and/or urine) and is preferably manufactured from a thin plastic film, although other flexible liquid impervious materials may also be used. Herein "flexible" refers to materials which are compliant and will readily conform to the general shape and contours of the human body. The backsheet prevents the exudates absorbed and contained in the absorbent core from wetting articles which contact the absorbent article such as bedsheets, pants, pajamas and undergarments. The backsheet may thus comprise a woven or nonwoven material, polymeric films such as thermoplastic films of polyethylene or polypropylene, or composite materials such as a film-coated nonwoven material. A suitable backsheet is a polyethylene film having a thickness of from about 0.012 mm (0.5 mil) to about 0.051 mm (2.0 mils). Exemplary polyethylene films are manufactured by Clopay Corporation of Cincinnati, Ohio, under the designation P18-1401 and by Tredegar Film Products of Terre Haute, Indiana, under the designation XP-39385. The backsheet may be embossed and/or matte finished to provide a more clothlike appearance. Further, the backsheet may permit vapors to escape from the absorbent core (i.e., the backsheet is breathable) while still preventing exudates from passing through the backsheet. The size of the backsheet is dictated by the size of the absorbent core and the exact absorbent article design selected.

The backsheet and the topsheet are positioned adjacent the garment surface and the body surface, respectively, of the absorbent core. The absorbent core is preferably joined with the topsheet, the backsheet, or both in any manner as is known by attachment means such as those well known in the art. However, embodiments of the present invention are envisioned wherein portions of the entire absorbent core are unattached to either the topsheet, the backsheet, or both.

For example, the topsheet and/or backsheet can be joined to the absorbent core or to each other in any suitable manner known in the art. The term "joined", as used in this specification, encompasses configurations in which an element is directly secured to another element by affixing the element directly to the other element; configurations in which the element is indirectly secured to the other element by affixing the element to intermediate member(s) which in turn are affixed to the other element; and configurations in which one element is integral with another element, i.e., one element is essentially part of the other element. The backsheet and/or the topsheet may be joined to the absorbent core or to each other by a uniform continuous layer of adhesive, a patterned layer of adhesive, or an array of separate lines, spirals, or spots of adhesive. Adhesives which have been found to be satisfactory are manufactured by H. B. Fuller Company of St. Paul, Minnesota under the designation HL-1258 or H-2031. The attachment means will preferably comprise an open pattern network of filaments of adhesive as is disclosed in U.S. Patent 4,573,986, issued to Minetola,

et al. on March 4, 1986. An exemplary attachment means of an open pattern network of filaments comprises several lines of adhesive filaments swirled into a spiral pattern such as illustrated by the apparatus and method shown in U.S. Patent 3,911,173 issued to Sprague, Jr. on October 7, 1975; U.S. Patent 4,785,996 issued to Zwieker, et al. on November 22, 1978; and U.S. Patent 4,842,666 issued to Werenicz on June 27, 1989. Alternatively, the attachment means may comprise heat bonds, pressure bonds, ultrasonic bonds, dynamic mechanical bonds, or any other suitable attachment means or combinations of these attachment means as are known in the art.

Skin care compositions are applied to at least a portion of the absorbent article. The skin care composition is applied to at least a portion of the body contacting surface and/or at least a portion of the body surface. Preferably, the skin care composition should be applied to a skin care zone to reduce skin disorders and/or wearer's discomfort and to provide skin care benefits. The amount of skin care composition applying to the skin care zone is selected such that the skin care zone delivers skin care benefits over the period of wearing the absorbent article. The skin care composition may be applied to a plurality of skin care zones of the absorbent article. The amount of the skin care composition may be different from a zone to a zone.

The skin care composition is also applied to the preferential acquisition zone. However, applying the skin care composition to the preferential acquisition zone may reduce absorbency of body exudatess at the preferential acquisition zone. Therefore, the amount of the skin care composition applying to the preferential acquisition zone is selected carefully in a balance between the skin care benefit that could be delivered by applying the skin care composition to the preferential acquisition zone and the absorbency through the preferential acquisition zone.

The skin care composition may be applied to the entirety of the body contacting surface including the skin care zone and the preferential acquisition zone. In this case, considering the characteristics of the preferential acquisition zone (i.e., effectively acquiring body exudatess) and the skin care zone (i.e., delivering skin care benefits), the skin care zone is provided with the skin care composition of greater basis weight than the preferential acquisition zone. The amount of the skin care composition in the skin care zone may vary from a zone to a zone as far as at least one skin care zone which should deliver skin care benefits has higher basis weight of the skin care composition than a portion of the preferential acquisition zone.

Further, the skin care composition may be applied to the garment surface of the absorbent article if desired. The skin care composition may be applied in any suitable configuration, such as a plurality of stripes, a plurality of wave lines, a plurality of dots, etc. Details of the skin care compositions are described below.

A preferred embodiment of an absorbent article is shown in FIG. 1. Shown in FIG. 1 is a sanitary napkin which is one type of absorbent articles and is used for external wear about the pudendal region of the wearer. As shown in FIG. 1, the sanitary napkin 20 preferably comprises an absorbent means (or "main body portion") 22, and two optional flaps 24. The sanitary napkin 20 has two surfaces, a body-facing surface or "body surface" or and a garment surface 20B. The sanitary napkin 20 is shown in FIG. 1 as viewed from its body surface 20A. The body surface 20A is intended to be worn adjacent to the wearer's body. The garment surface 20B is intended to be placed adjacent to the wearer's undergarments when the sanitary napkin 20 is worn. In the embodiment shown in FIG. 1, "body contacting surface" includes the body surface 20A.

The sanitary napkin 20 has two centerlines, a principal longitudinal centerline L and a principal transverse centerline T. The term "longitudinal", as used herein, refers to a line, axis or direction in the plane of the sanitary napkin 20 that is generally aligned with (e.g., approximately parallel to) a vertical plane which bisects a standing wearer into left and right body halves when the sanitary napkin 20 is worn. The terms "transverse" or "lateral" used herein, are interchangeable, and refer to a line, axis or direction which lies within the plane of the sanitary napkin 20 that is generally perpendicular to the longitudinal direction.

FIG. 1 shows that the main body portion 22 of the sanitary napkin 20 comprises the portion of the sanitary napkin without the flaps 24. The main body portion 22 has two spaced apart longitudinal edges 26, two spaced apart transverse or end edges (or "ends") 28, which together form the periphery 30 of the main body portion. The main body portion 22 also has two end sections, which are designated first end section (i.e., front section) 32 and second end section (i.e., back section) 34. A central section 36 is disposed between the end sections 32 and 34. The end sections 32 and 34 extend outwardly in the longitudinal direction from the edges of the central section 36 about 1/8 to about 1/3 of the length of the main body portion. A detailed description of the characteristics of a central section and two end sections for a sanitary napkin is contained in U.S. Patent 4,690,680 issued to Higgins on September 1, 1987.

The main body portion 22 of the sanitary napkin 20 can be of any thickness, including relatively thick, intermediate thickness, relatively thin, or even very thin (or "ultra thin"). An "ultra-thin" sanitary napkin 20 as described in U.S. Patents 4,950,264 and 5,009,653 issued to Osborn preferably has a caliper of less than about 3 millimeters. The embodiment of the sanitary napkin 20 shown in the drawings is intended to be an example of an ultra-thin sanitary napkin. The main body portion 22 of the sanitary napkin 20 may also be relatively flexible, so that it is comfortable for the wearer. It should, however, be understood that the sanitary napkin shown is merely one embodiment, and that the present invention is not limited to absorbent articles of the type or having the specific configurations shown in the drawings.

FIG. 2 shows the individual components of the main body portion 22 of the sanitary napkin 20. The main body

portion 22 of the sanitary napkin 20 preferably comprises at least three primary components. These include a liquid pervious topsheet 38, a liquid impervious backsheet 40, and an absorbent core 42 positioned between the topsheet 38 and the backsheet 40. Optionally, the absorbent core 42 may include other components such as an acquisition component 44. The acquisition component 44 may either be a separate component positioned between the topsheet 38 and the absorbent core 42, or it may comprise part of a composite topsheet or part of the absorbent core 42.

The sanitary napkin 20 has a preferential acquisition zone and a skin care zone. FIG. 1 shows examples of the preferential acquisition zone 25 and the skin care zone 27. The preferential acquisition zone 25 positions generally at the center of the sanitary napkin 20 such that the preferential acquisition zone 25 includes at least a portion of the sanitary napkin 20 along the longitudinal centerline L and at least a portion of the central section 36. The skin care zone 27 positions generally at a portion of the periphery of the preferential acquisition zone 25 such that the skin care zone 27 includes at least a portion of the sanitary napkin 20 transversely or longitudinally outside the preferential acquisition zone 25. Suitable configurations of the preferential acquisition zone 25 include, but are not limited to, oval shape, race-track shape, or circle shape. Alternatively, the configuration of the preferential acquisition zone 25 may have rectangle shape, triangle shape, or polygon shape, or any other shapes which is suitable as a preferential acquisition zone to effectively acquire body fluid at the source of the body fluid. The skin care zone 27 may be the remainder of the sanitary napkin 20 other than the preferential acquisition zone 25. However, the skin care zone 27 may be only a portion of the sanitary napkin 20 transversely and/or longitudinally outside the preferential acquisition zone 25. In the embodiment shown in FIG. 1, the preferential acquisition zone 25 has an oval shape and positions generally at the center in the longitudinal direction and in the transverse direction of the sanitary napkin 20. The major axis of the oval shape of the preferential acquisition zone 25 is disposed to align with the longitudinal centerline L of the sanitary napkin 20. The skin care zones 27 positions at the first end section 32 and the second end section 34.

The preferential acquisition zone 25 is preferably placed in a specific positional relationship with respect to the area of typical body fluid deposition of the sanitary napkin 20 and with respect to a specific portion of the wearer's body where body fluid is discharged. Thus, the preferential acquisition zone 25 is placed in the vicinity of the point of discharge of body fluid so as to be capable of rapidly acquiring a majority of body fluid at their contact zone. It is preferable that the preferential acquisition zone 25 covers at least a portion of the vulva of the wearer when the sanitary napkin is applied on the wearer's body. The preferential acquisition zone 25 does not necessarily contact the portion of the vulva of the wearer. However, it is preferable to contact the portion of the vulva to absorb body fluid directly from the wearer's body into the preferential acquisition zone.

The preferential acquisition zone 25 may cover portions of the interior surfaces of the wearer's labia minora such that the preferential acquisition zone fits interlabially between the interior surfaces of the wearer's labia minora. The preferential acquisition zone 25 may cover substantially all of the interior surfaces of the wearer's labia minora up to and including contacting and covering the floor of the wearer's vestibule. The preferential acquisition zone 25 may cover portions of the exterior surfaces of the wearer's labia minora. The preferential acquisition zone 25 may cover portions of both the interior surface and the exterior surface of the wearer's labia minora. The preferential acquisition zone 25 may cover a portion of the surface of the wearer's labia majora.

The sanitary napkin 20 preferably acquires greater than or equal to 50 %, preferably greater than or equal to 60 %, more preferably greater than or equal to 70 %, of body fluid absorbed by the sanitary napkin 20, through the preferential acquisition zone 25. Preferably, the sanitary napkin 20 acquires almost all the body fluid through the preferential acquisition zone 25 when the sanitary napkin 20 is placed in a right position with the wearer's body. Since body fluid is sometimes discharged in gushes, the preferential acquisition zone 25 should be able to rapidly acquire and transport body fluid from the wearer's body to an element of the sanitary napkin 20, such as preferably to the absorbent component 32 with a minimum of body fluid flow resistance.

In one embodiment shown in FIG. 1, the preferential acquisition zone 25 has an oval shape. The preferential acquisition zone 25 has a dimension to cover the wearer's vulva. The preferential acquisition zone 25 has a longitudinal length of less than or equal to about 120 mm, preferably less than or equal to about 110 mm, more preferably less than or equal to about 100 mm, and of more than or equal to about 20 mm, preferably more than or equal to about 30 mm, more preferably more than or equal to about 40 mm. The preferential acquisition zone 25 may have a transverse width of less than or equal to about 80 mm, preferably less than or equal to about 70 mm, more preferably less than or equal to about 60 mm, and of more than or equal to about 10 mm, preferably more than or equal to about 20 mm, more preferably more than or equal to about 30 mm.

The preferential acquisition zone 25 also may be so flexible that the preferential acquisition zone 25 maintains sustained contact with a portion of the surface of the wearer's labia minora. The sustained contact secures the preferential acquisition zone to acquire body fluid without leakage or with minimal leakage of body fluid outside the preferential acquisition zone 25 over a period of use of the sanitary napkin.

The skin care composition applied to the central area 96 of the sanitary napkin 20 tends to reduce adherence of the sanitary napkin to the wearer's skin at the central section of the sanitary napkin 20 (which corresponds to the portion of the wearer's body around vulva).

The skin care zone 27 is preferably placed in a specific positional relationship with respect to the area in the sanitary napkin 20 where body fluid is not typically deposited and with respect to a portion of the wearer's body where body fluid is not typically discharged, when the sanitary napkin 20 is placed in a right place with the wearer's body. The skin care zone 27 is expected to provide skin care benefits to the wearer through the transfer of the skin care composition applied on the skin care zone 27.

The skin care zone 27 of the sanitary napkin 20 may cover at least a portion of the surface of the wearer's labia majora. The skin care zone 27 may cover all the surface of the wearer's labia majora. Further, the skin care zone may cover a portion of the surface of the wearers body outside the labia minora. When the preferential acquisition zone 25 covers only a portion of the surface of the wearer's labia minora, the skin care zone 27 may extend into a portion of the wearer's labia minora (though it is less preferable).

The skin care zone 27 does not directly contact a portion of the wearer's body where body fluid is discharged when the sanitary napkin 20 is placed in a right position with the wearer's body. Body fluid is not typically deposited on the skin care zone 27. This reduces the opportunity that body fluid is absorbed into the absorbent component 32 at the skin care zone 27. Therefore, the skin care composition may be applied to the skin care zone 27 without compromising the absorbency of body exudatess.

FIGS. 4 and 5 show examples of the skin care zone. In FIG. 4, the preferential acquisition zone 25 positions at the center of the body surface. The skin care zone 27 includes the remainder of the body contacting surface other than the preferential acquisition zone 25. Alternatively, as shown in FIG. 5, the skin care zone may be a portion of the remainder of the body contacting surface other than the preferential acquisition zone 25. Preferably, the skin care zone includes at least one selected from the group consisting of a front skin care zone 102, a back skin care zone 104, a periphery skin care zone 106, and a flap skin care zone 108. Preferably, the skin care zone includes all of them.

The front skin care zone 102 includes at least a portion of the front section 32 of the sanitary napkin 20. The front skin care zone 102 is the portion which comes in contact with the wearer's body around pubic hairs. The front skin care zone 102 provides skin care benefits to the wearer through the transfer of the skin care composition applied on the front skin care zone 102. For example, the skin care composition applied on the front skin care zone 102 reduces itch at the front section 32 of the sanitary napkin 20.

The back skin care zone 104 includes at least a portion of the back section 34 of the sanitary napkin 20. The back skin care zone 104 is the portion which comes in contact with the wearer's body around anus or perineum. The back skin care zone 104 provides skin care benefits to the wearer through the transfer of the skin care composition applied on the back skin care zone 104. For example, the skin care composition applied on the back skin care zone 104 reduces adherence of the topsheet of the sanitary napkin to the wearer's body.

The periphery skin care zone 106 includes at least a portion of the periphery portion of the sanitary napkin 20 contiguous with the periphery 30. Preferably, the periphery skin care zone 106 includes a front periphery portion 110 and a back periphery portion 112, both of which come in contact with the wearer's body. Typically, the front periphery portion 110 and the back periphery portion 112 becomes stiff because the topsheet 38 and backsheet 40 are joined there. The stiff front and back periphery portions 110 and 112 causes the wearer, e.g., the discomfort such as itch and/or abrasion due to rubbing of the portions to the wearer's body. This is more obvious when the topsheet 38 and the backsheet 40 are densified by heat, pressure, or mixtures thereof. The periphery skin care zone 106 provides skin care benefits to the wearer through the transfer of the skin care composition applied on the periphery skin care zone 106. For example, the skin care composition applied on the periphery skin care zone 106 reduces the wearer's discomfort such as itch and/or abrasion associated due to rubbing of the periphery portion of the sanitary napkin 20 with the wearer's body.

The flap skin care zone 108 includes at least a portion of the flap of the sanitary napkin 20. The flap skin care zone 108 includes a portion of the flap 24 (which is explained below in detail) which positions laterally outwardly of the proximal edge 60 of the flap 24, and a portion of the main body portion 22 which positions laterally inwardly of the proximal edge 60 of the flap 24. Preferably, the flap skin care zone 108 includes a flap folding portion where the flap 24 is expected to be folded when the sanitary napkin 20 is used. The flap skin care zone 108 provides skin care benefits to the wearer through the transfer of the skin care composition applied on the flap skin care zone 108. For example, the skin care composition applied on the flap skin care zone 108 reduces the wearer's discomfort such as itch and/or abrasion associated due to rubbing of the flap folding portion of the sanitary napkin 20 with the wearer's body.

Other skin care zones are possible. If the sanitary napkin 20 has cuffs along the longitudinal side edges 26, the skin care zone may includes the surface of the cuffs which is treated with the skin care composition. Further, the skin care zone may include a portion of the garment surface of the sanitary napkin 20 which is treated with the skin care composition.

A topsheet 38 which is particularly suitable for use in the sanitary napkin 20 comprises an apertured formed film. Apertured formed films are preferred for the topsheet because they are pervious to body exudates and, if properly apertured, have a reduced tendency to allow liquids to pass back through and rewet the wearer's skin. Thus, the surface of the formed film which is in contact with the body remains dry, thereby reducing body soiling and creating a more comfortable feel for the wearer. Suitable formed films are described in U.S. Patent 3,929,135, entitled "Absorptive

Structures Having Tapered Capillaries", which issued to Thompson on December 30, 1975; U.S. Patent 4,324,246 entitled "Disposable Absorbent Article Having A Stain Resistant Topsheet", which issued to Mullane, et al. on April 13, 1982; U.S. Patent 4,342,314 entitled "Resilient Plastic Web Exhibiting Fiber-Like Properties", which issued to Radel, et al. on August 3, 1982; U.S. Patent 4,463,045 entitled "Macroscopically Expanded Three-Dimensional Plastic Web Exhibiting Non-Glossy Visible Surface and Cloth-Like Tactile Impression", which issued to Ahr et al. on July 31, 1984; U.S. Patent 4,780,352 entitled "Covering Structure For Absorbent Hygienic Sanitary Products, and an Absorbent Product Having Such A Covering", which issued to Palumbo on October 25, 1988; U.S. Patent 5,006,394 "Multilayer Polymeric Film" issued to Baird on April 9, 1991; and U.S. Patent Application Serial No. 08/442,935 entitled "Fluid Transport Webs Exhibiting Surface Energy Gradients" filed in the name of Ouellette, et al. on May 31, 1995 (PCT Publication WO 96/00548, published January 11, 1996).

In a preferred embodiment, the topsheet 38 comprises an apertured formed film made in accordance with U.S. Patents 4,342,314 issued to Radel, et al. and 4,463,045 issued to Ahr, et al., which is marketed on sanitary napkins as the DRI-WEAVE topsheet by The Procter & Gamble Company of Cincinnati, OH. Such an apertured film is preferably obtained as product No. X-5652 from Tredegar Film Products of Terre Haute, IN. In this preferred embodiment, during manufacture the resin used to form the apertured film is preferably provided with a surfactant incorporated therein.

In preferred embodiments of the present invention, the body surface of the topsheet 38 is hydrophilic so that liquids will be transferred through the topsheet more readily. If the topsheet is made of a hydrophobic material, at least the upper surface of the topsheet is treated to be hydrophilic so that liquids will transfer through the topsheet more rapidly. This diminishes the likelihood that menstrual fluid will flow off the topsheet rather than flowing into and being absorbed by the absorbent core. The body surface of the topsheet 38 can be made hydrophilic by treating it with a surfactant. Suitable methods of treating a topsheet with a surfactant are described in U.S. Patent 4,950,254 issued to Osborn; U.S. Patents 4,988,344 entitled "Absorbent Articles with Multiple Layer Absorbent Layers" issued to Reising, et al on January 29, 1991; and U.S. Patent 4,988,345 entitled "Absorbent Articles with Rapid Acquiring Absorbent Cores" issued to Reising on January 29, 1991.

A backsheet 40 which is particularly suitable for use in the sanitary napkin 20 comprises a polyethylene film having a thickness of from about 0.012 mm (0.5 mil) to about 0.051 mm (2.0 mils) as stated above. A suitable backsheet material is obtained as product No. 18-1401 from the Clopay Corporation of Cincinnati, Ohio. A suitable breathable backsheet material is a laminate of an apertured film such as that described in U.S. Patent 3,929,135 issued to Thompson which is inverted so that the smaller openings of the tapered capillaries face the absorbent core 42 which is adhesively laminated to a microporous film such as that described in Exxon's U.S. Patent 4,777,073.

An absorbent core 42 which is particularly suitable for use in the sanitary napkin 20 comprises a multi-bonded air laid nonwoven material. In this preferred embodiment, this multi-bonded air laid nonwoven material comprises about 52% cellulose fibers, about 20% bi-component fibers, about 25% superabsorbent hydrogel-forming material (or absorbent gelling material) particles, and about 3% latex binder. The absorbent core 42 preferably has a basis weight of about 125 g/yd$^2$ (about 150 g/m$^2$), including the particles of absorbent gelling material. Preferably, this multi-bonded air laid nonwoven absorbent core 42 is formed by depositing three streams of cellulose and bi-component fibers, with absorbent gelling material particles 58 laid down with the last stream of fibers to form the bottom portion of the absorbent core. While the absorbent core 42 is shown as a laminate in FIGS. 2 and 3, in preferred embodiments, the fibers are blended together to form a single web. Such a multi-bonded air laid nonwoven material is preferably obtained in roll form as product 915000X313 from Merfin Hygienic Products.

In alternative embodiments, the multi-bonded air laid nonwoven material used for the absorbent core can be bonded using some material other than latex (such as starch or PVA, for example). In another alternative embodiment, the absorbent core can be formed as a laminate that preferably also has a basis weight of about 150 g/m$^2$ and comprises two (or more) layers of multi-bonded air laid nonwoven material with the particles of absorbent gelling material therebetween. Suitable laminate absorbent core structures are described generally in U.S. Patents 4,950,264 and 5,009,653 issued to Osborn, U.S. Patent 5,460,623 issued to Emenaker, et al. Another suitable absorbent core is described in U.S. Patent Application Serial No. 08/122,114, entitled "Sanitary Napkin Having Core Predisposed To A Convex Upward Configuration", filed in the name of Hines, et al. on September 16, 1993 (PCT Publication No. WO 95/07674, published March 23, 1995).

In the case of thicker sanitary napkins, the absorbent core 42 is preferably comprised of airfelt. Suitable absorbent cores for thicker sanitary napkins are described in U.S. Patent 5,234,422 issued to Sneller, et al. In a preferred embodiment, the topsheet 38, acquisition component 44, and absorbent core 42 can be provided with embossed channels as shown in the Sneller, et al. patent. If such embossed channels are used, they preferably lie laterally outside of the longitudinally-oriented concave lines 56A defining the sides of the unbonded window 54.

The absorbent core 42 optionally includes an acquisition component 44. The acquisition component 44 can be made from any materials suitable. The acquisition component 44 may, for example, be comprised of woven or nonwoven materials. The fibers or other components of these materials may be synthetic, or partially synthetic and partially natural. Suitable synthetic fibers include polyester, polypropylene, polyethylene, nylon, viscous rayon, or cellulose acetate fibers. Suitable natural fibers include cotton, cellulose, or other natural fibers. The acquisition component 44 may also be at least partially comprised of cross-linked cellulose fibers. The acquisition component 44, if nonwoven, can be made by a number of different processes. These include, but are not limited to: air laid, wet laid, meltblown, spunbonded, carded, thermally bonded, air-through bonded, powder bonded, latex bonded, solvent bonded, spunlaced, and combinations of the foregoing.

An acquisition component 44 which is particularly suitable for use in the sanitary napkin 20 comprises a laminate of two nonwoven materials. The uppermost layer preferably comprises an 19 g/yd$^2$ (22.5 g/m$^2$) spunbonded polypropylene nonwoven material referred to as product No. 065MLPV60U (or "P-9") obtained from Fiberweb, North America of Washougal, WA. The underlying layer preferably comprises a multi-bonded air laid nonwoven material that is thermally bonded using powder bonding and latex bonding. In a preferred embodiment, this multi-bonded air laid nonwoven material comprises about 77% cellulose fibers, about 20% powder binder, and about 3% latex binder (1.5% sprayed on each side of the web) and has a basis weight of about 50 g/yd$^2$ (about 60 g/m$^2$). Such a multi-bonded air laid nonwoven is preferably obtained as product No. 90830X312 from Merfin Hygienic Products, Ltd. of Delta, British Columbia, Canada. These two nonwoven layers are preferably laminated together by depositing the multi-bonded air laid nonwoven material on the spunbonded polypropylene nonwoven material. The spunbonded material is used as a process aid or carrier web in the process of forming this laminate.

In alternative embodiments, the spunbonded polypropylene nonwoven material may have a greater or a lower basis weight, or it may be replaced by an air laid tissue, a wet laid tissue, or any of the materials described above. If a wet laid tissue is used instead of a polypropylene nonwoven material, the orientation of the laminate is preferably reversed so that in the finished product, the multi-bonded air laid nonwoven material lies above the wet laid tissue layer. In the case of thicker sanitary napkins, any of the acquisition components described above can be used. Additionally, in one preferred thicker sanitary napkin embodiment, a low density latex bonded air laid material can be used as the entire acquisition component (that is, no tertiary topsheet is required). A low density latex bonded air laid material suitable for this purpose is a material having a basis weight of about 80 g/m$^2$ known as product No. FG413MHB, which is obtained from Walkisoft, USA of Mt. Holly, NC.

The topsheet 38, the backsheet 40, and the absorbent core 42 may be assembled in a variety of configurations known in the art (including layered or "sandwich" configurations and wrapped or "tube" configurations). FIGS. 1-3 show a preferred embodiment of the sanitary napkin 20 assembled in a sandwich construction. In FIGS. 1-3, the topsheet 38 and the backsheet 40 have length and width dimensions generally larger than those of the absorbent core 42. The topsheet 38 and the backsheet 40 extend beyond the edges of the absorbent core 42 to form portions of the periphery 30. In the embodiment shown in FIGS. 2 and 3 having the acquisition component 44, the garment-facing side of the topsheet 38 is preferably joined to the body-facing side of the absorbent core (i.e., the body-facing side of the acquisition component 44). If the absorbent core 42 has a layered structure, each layer may be joined each other, if desired. The acquisition component 44 may be joined to the absorbent core 42, if desired. If these components are joined, they can be joined in any of the manners described hereinabove. The backsheet 40 is preferably joined to the garment-facing side of the absorbent core 42 by adhesives.

The portions of the topsheet 38 and backsheet 40 that extend beyond the edges of the absorbent core 42 are preferably also joined to each other. Preferably, in the embodiment shown, these portions of the topsheet 38 and backsheet 40 are joined using adhesives over substantially the entire portions that extend beyond the edges of the absorbent core 42 and a crimp seal at the end edges 28 of the main body portion 22 where the topsheet 38 and backsheet 40 are densified by the application of pressure or heat and pressure.

The sanitary napkin 20 shown in FIGS. 1-3, as discussed above, comprises an optional pair of flaps 24 that are joined to the main body portion 22. The flaps 24 extend laterally outward beyond the longitudinal side edges 26 of the main body portion 22 from their proximal edges 60 to their distal edges (or "free end") 62. The flaps 24 extend outward from at least the central section 36 of the main body portion 22.

The flaps 24 can be joined to the main body portion 22 in any suitable manner. Preferably, in the embodiment shown in FIGS. 1-3, the flaps 24 are integral with the main body portion 22 (that is, the flaps 24 comprise integral extensions of the topsheet 38 and backsheet 40). In other alternative embodiments, the flaps 24 can comprise separate components that are joined to the main body portion 22. The flaps 24 are each joined to (or associated with) main body portion 22 along a juncture. This is typically a longitudinally-oriented (or "longitudinal") juncture, such as lines of juncture 68. As used herein, the terms "juncture" (or "line of juncture") refer to regions where the flaps 24 extend from or are joined to the main body portion 22. The line of juncture 68 in the embodiment illustrated in the drawings can be considered to be defined by concave inwardly-oriented regions or lines. When the sanitary napkin 20 is worn by the wearer, the flaps 24 are folded under the wearers undergarment. The flaps 24 are typically folded along or adjacent the proximal edges 60. If the width of crotch of wearer’s undergarment is narrower than that of the main body portion 22, the flaps 24 may be folded along a longitudinal portion of the main body portion 22 inside the proximal edge 60. If the width of crotch of wearer’s undergarment is wider than that of the main body portion 22, the flaps 24 may be folded along a hinge 70 of the main body portion 22 which is described below.

The flaps 24 can be in any suitable configuration. Suitable flaps are described in Reexamined Patent No. B1 4,589,876 entitled "Sanitary Napkin", issued to Van Tilburg, Certificate of Reexamination issued April 27, 1993; U.S. Patent 4,687,478 entitled "Shaped Sanitary Napkin With Flaps", which issued to Van Tilburg on August 18, 1987; U.S. Patent 5,389,094 entitled "Absorbent Article Having Flaps and Zones of Differential Extensibility" issued to Lavash, et al. on February 14, 1995; U.S. Patent 5,558,663 entitled "Absorbent Article Having Undergarment Covering Components With Zones of Extensibility" issued to Weinberger, et al. on September 24, 1996 (which describes an alternative to flaps that are applied by the wearer); and in International Patent Application Serial No. PCT US 96/15957 entitled "Absorbent Article Having Flaps With Step Configuration and Zones of Extensibility" filed on October 3, 1996, in the name of Lash, et al.

The garment surface 20B of the sanitary napkin 20 may include, and preferably does include, fasteners for attaching the sanitary napkin to the wearer's undergarment. FIG. 2 shows the central pad fastener 82 which is adapted to secure the main body portion 22 of the sanitary napkin to the crotch region of an undergarment. Any types of fasteners known in the art, such as adhesive fasteners and mechanical fasteners can be used. Fasteners comprising adhesives have been found to work well for this purpose, with pressure-sensitive adhesives being preferred. In a preferred embodiment, the central pad fastener 82 comprises a pair of spaced apart longitudinally-oriented strips or zones of adhesive that are centered about the longitudinal centerline L.

The outer surface of the flaps 24, adjacent the distal edges 62 of the flaps, is preferably provided with a flap adhesive 84. The flap adhesive 84 is used to assist in maintaining the flaps 24 in position after they are wrapped around the edge of the crotch portion of the panty. Suitable adhesive fasteners are described in greater detail in U.S. Patent 4,917,697. The flaps 24 can be maintained in position by attaching the flaps 24 to the undergarment, or to the opposing flap.

The fasteners are not limited to adhesive attachment means. Any type of fastener used in the art can be used for such purpose. For example, the sanitary napkin 20 could be secured to the wearer's undergarment by mechanical fasteners, such as VELCRO, or the fasteners described in U.S. Patent 4,946,527 entitled "Pressure-Sensitive Adhesive Fastener and Method of Making the Same" issued to Battrell on August 7, 1990, or U.S. Patent 5,392,498 entitled "Non-Abrasive Skin Friendly Mechanical Fastening System" issued to Goulait, et al. on February 28, 1995. For simplicity, however, the fasteners will be described in terms of adhesive attachment means.

The adhesive attachment means are respectively covered by removable release liners, central pad release liner and flap release liner, both designated 86. The pressure-sensitive adhesives should be covered with release liners 86 to keep the adhesives from sticking to extraneous surfaces prior to use. Suitable release liners are described in U.S. Patent 4,917,697. A particularly preferred release liner which also serves as an individual package for wrapping the sanitary napkin is described in U.S. Patent 4,556,146 issued to Swanson, et al.

The sanitary napkin 20 shown in FIGS. 1-3 may have a deformed region that forms a hinge 70 between the main body portion 22 and at least a portion of the flaps 24. The sanitary napkin 20 preferably also has at least one zone of extensibility (or "zone of differential extensibility") 72 for relieving the stresses on the flaps 24 when they are folded around a panty crotch. These are described in PCT publication WO 97/12576 published on April 10, 1997 titled "Absorbent Article Having Flaps With A Deformed Hinge And Zones Of Extensibility".

Skin care compositions are applied to the skin care zone. Skin care compositions are also applied to the preferential acquisition zone. The amount of the skin care composition applied on each zone should be selected such that each zone serves expected functions. The preferential acquisition zone is primarily expected to absorb body exudatess fairly quickly while the preferential acquisition zone may provide skin care benefits to the wearer. Therefore, it is preferable that the relatively small amount of the skin care composition, which may hinder the preferential acquisition zone from absorbing bodily fluid, is applied on the preferential acquisition zone. On the other hand, the skin care zone is primarily expected to provide skin care benefits to the wearer while the skin care zone may be able to absorb body exudatess therethrough. Therefore, it is preferable that the relatively large amount of the skin care composition is applied on the skin care zone.

It is preferable that the skin care zone is provided with the skin care composition of greater basis weight that the preferential acquisition zone. The skin care zone is provided with the skin care composition of between 0.1 g/m$^2$ and 100 g/m$^2$, preferably 0.5 g/m$^2$ and 90 g/m$^2$, more preferably 1 g/m$^2$ and 80 g/m$^2$. The preferential acquisition zone is provided with the skin care composition of not greater than 20 g/m$^2$, preferably not greater than 12 g/m$^2$, more preferably 4 g/m$^2$. The preferential acquisition zone may not be provided with the skin care composition. The preferential acquisition zone and the skin care acquisition zone may be provided with any amount of the skin care composition selected from the above range. However, it is important that skin care zone is provided with the skin care composition of greater basis weight that the preferential acquisition zone such that each zone achieves its expected functions.

When the skin care composition is applied on a plurality of skin care zones, the amount of one skin care zone may be different from that of the other skin care zones. Alternatively, the amount of the skin care composition on each skin care zone may be different. For example, if the skin care composition is applied on the front skin care zone 102, the back skin care zone 104, the periphery skin care zone 106, and the flap skin care zone 108, the flap skin care zone 108 may be provided with the skin care composition of greater basis weight than the other skin care zones. Alternatively, the periphery skin care zone 106 may be provided with the skin care composition of greater basis weight than the other

skin care zones. Alternatively, each of the front skin care zone 102, the back skin care zone 104, the periphery skin care zone 106, and the flap skin care zone 108 may be provided with a different amount of the skin care composition. Thus, the amount of the skin care composition on each skin care zone may be selected based on the expected function of each skin care zone. Further, each skin care zone may be provided with the different type of skin care compositions.

The skin care composition is applied on the topsheet of the preferential acquisition zone. In order to maintain the acquisition performance at the preferential acquisition zone, the amount of the skin care composition is selected such that acquisition rate of the portion of the topsheet which is provided with the skin care composition is not less than 70 % of the acquisition rate of the topsheet before the skin care composition is applied thereon, preferably not less than 75 % of the acquisition rate of the topsheet before the skin care composition is applied thereon, more preferably not less than 85 % of the acquisition rate of the topsheet before the skin care composition is applied thereon. The acquisition rate of the portion of the topsheet can be measured by the following method. The method includes the steps; (1) prepare a weight plate (100mm of length x 100mm of width x 30mm of thickness, 0.25psi) which has a hole (diameter 25.4mm) at the center on the plate, (2) put the weight plate such that the hole positions on the portion of the topsheet to be measured which is at the center of the absorbent article, (3) apply 10ml of sheep blood to the absorbent article through the hole of the weight plate, (4) measure time after the sheep blood is applied until the sheep blood is completely drained in the hole of the weight plate (acquisition time), and (5) calculate the acquisition rate as shown below;

$$\text{Acquisition Rate (ml/second)} = 10\text{ml}/\ \text{Acquisition Time (second)}$$

Numerous other embodiments of the sanitary napkin 20 are possible. For example, the main body portion of the sanitary napkin can be provided in the form of a compound sanitary napkin that has its components bonded as described herein for improved integrity and acquisition. General descriptions of compound sanitary napkins are found in P&G's U.S. Patent 4,425,130 entitled "Compound Sanitary Napkin" issued to DesMarais, et al. on January 10, 1984, and in Statutory Invention Registration H1614 entitled "Body Fitting Compound Sanitary Napkin", published in the name of Mayer, et al. on November 5, 1996. To form the compound sanitary napkin, a sanitary napkin such as that described herein can serve as the panty protector (or "base pad") and a tube of absorbent material wrapped by a topsheet (or "primary menstrual pad") can be placed on top of the sanitary napkin and attached thereto at the ends. The fusion bonding on the base pad is preferably distributed in the same manner as shown on the drawings herein. The attachment of the tube to the sanitary napkin is preferably achieved by fusion bonding extensions of the topsheet material at the ends of the tube to the base pad. In some preferred embodiments of such a compound sanitary napkin, there may also be attachment between the ends of the tube of absorbent material and the base pad. The tube of the compound sanitary napkin can be attached to the base pad between its ends by any suitable attachment means, such as by adhesives.

The skin care composition may be applied to the body contacting surface of other types of feminine hygiene absorbent articles. Such absorbent articles having a body contacting surface include interlabial absorbent articles, panti-liners, incontinence articles.

Herein "tampon" refers to any type of absorbent structure which is inserted into the vaginal canal or other body cavities for the absorption of fluid therefrom. The basic tampon structures are described in U.S. Patents 1,926,900 issued to Haas on September 12, 1933; U.S. Patent 1,946,911 issued to Haas on July 3, 1934; and U.S. Patent 3,322,123 issued to Giswold, et al. on May 30, 1967.

Herein "interlabial absorbent article" refers to an absorbent device that is insertable into the interlabial space of a female wearer for catamenial purposes, incontinence protection, or both. Suitable interlabial absorbent articles are disclosed in, e.g., U.S. Patent 5,762,644 entitled "Toilet-Disposable Absorbent Interlabial Device" issued to Osborn, et al. on June 9, 1998; PCT Publication No. WO 98/29078 entitled "Thin Comfortable Interlabial Absorbent Structure" published in the name of Osborn, et al. on July 9, 1998; U.S. Patent Des. 404,814 entitled "Interlabial Absorbent Device" issued to Mayer on January 26, 1999; U. S. Application Serial No. 09/071,425, filed on May 1, 1998 in the name of Brown, et al.

The terms "panty liner" or "panti-liner" refer to absorbent articles that are less bulky than sanitary napkins which are generally worn by women between their menstrual periods. Suitable absorbent articles are disclosed in, e.g., U.S. Pat. No. 4,738,676 entitled "Pantiliner" issued to Osborn on Apr. 19, 1988.

Herein "incontinence article" refers to pads, undergarments (pads held in place by a suspension system of same type, such as a belt, or the like), inserts for absorbent articles, capacity boosters for absorbent articles, briefs, bed pads, and the like regardless of whether they are worn by adults or other incontinent persons. Suitable incontinence articles are disclosed in, e.g., U.S. Patent No. 4,253,461 issued to Strickland, et al. on March 3, 1981; U.S. Patent Nos. 4,597,760 and 4,597,761 issued to Buell; the above-mentioned U.S. Patent No. 4,704,115; U.S. Patent No. 4,909,802 issued to Ahr, et al.; U.S. Patent No. 4,964,860 issued to Gipson, et al. on October 23, 1990; and in U.S. Patent Application Serial No. 07/637,090 filed by Noel, et al. on January 3, 1991 (PCT Publication No. WO 92/11830 published on July

23,1992).

The skin care composition may be applied to a portion of or the entirety of the body contacting surface of absorbent articles. For example, the skin care composition may be applied on a portion of or the entirety of the topsheet of the absorbent articles.

Representative topsheets treated with a skin care composition are described in, e.g., U.S. Patent 5,643,588, "Diaper Having a Lotioned Topsheet", issued to Roe, Bakes & Warner on July 1, 1997; and U.S. Patent 5,635,191, "Diaper Having a Lotioned Topsheet Containing a Polysiloxane Emollient", issued to Roe & Mackey on June 3, 1997; U.S. Patent 5,609,587, "Diaper Having a Lotioned Topsheet Comprising a Liquid Polyol Polyester Emollient and an Immobilizing Agent", issued to Roe on March 11, 1997; and U.S. Patent 5,607,760, "Disposable Absorbent Article Having a Lotioned Topsheet Containing an Emollient and a Polyol Polyester Immobilizing Agent", issued to Roe, on March 4, 1997. Methods for delivering a skin care composition via the repeated use of absorbent articles having such treated topsheets are disclosed in U.S. Patent Application Serial No. 08/926,532 "A Method For Maintaining or Improving Skin Health", Elder, et al., filed on September 10, 1997; U.S. Patent Application Serial No. 08/926,533 "A Method For Improving Skin Condition", Van Rijswijck, et al. filed on September 10, 1997; and U.S. Patent Application Serial No. 08/908,852 "Diaper Having A Lotioned Topsheet", Roe, et al. filed on August 8, 1997. Representative cuffs treated with a skin care composition are described in, e.g., U.S. Patent Application Serial No. 08/766,386 "Absorbent Articles Having Lotioned Leg Cuffs", Schulte et al, filed on December 3, 1996; U.S. Patent Application Serial No. 08/840,039 "Absorbent Articles Having Lotioned Leg Cuffs Containing a Polysiloxane Emollient", Schulte et al, filed on April 24, 1997; U.S. Patent Application Serial No. 08/962,310 "Absorbent Article Having cuffs and Topsheet with Skin Care Composition Disposed Thereon", Schulte et al, filed on October 31, 1997; U.S. Patent Application Serial No. 08/962,312 "Absorbent Article Having cuffs and Topsheet with Skin Care Composition(s) Disposed Thereon", Vanri et al, filed on October 31, 1997; each of which is incorporated herein by reference. Representative interlabial absorbent articles treated with a skin care composition are disclosed in, e.g., U.S. Patent Application Serial No. 08/869,897 "Emollient-Treated Absorbent Interlabial Device", Osborn et al, filed on June 5, 1997; U.S. Patent Application Serial No. 08/869,700 "Absorbent Interlabial Device Treated With A Polysiloxane Emollient", Osborn et al, filed on June 5, 1997. Representative absorbent articles having breathability treated with a skin care composition are disclosed in, e.g., U.S. Patent Application Serial No. 08/926,566 "Disposable Absorbent Articles Providing a Skin Condition Benefit", ELDER et al., filed on 09/10/97.

B. Skin Care Composition.

The skin care composition(s) preferably delivers skin effects. It is preferred that the skin care composition should provide a protective, nonocclusive function (e.g., a relatively liquid impervious but vapor pervious barrier) to avoid skin hyperhydration and skin exposure to materials contained in body exudates; an abrasion minimizing function to reduce skin irritation in the areas where the body contacting surface of absorbent articles contact the wearer's skin; or contain agents that deliver, either directly or indirectly, skin care benefits. For example, indirect benefits include improved removal of skin irritants such as menses, feces or urine. The composition may be in a variety of forms, including, but not limited to, emulsions, lotions, creams, ointments, salves, powders, suspensions, encapsulations, gels, and the like.

The skin care compositions may be solid or semisolid at 20°C and are solid or semisolid at 40°C. Herein, "semisolid" means the skin care composition has a viscoelastic property (i.e., when no shear stress is applied, the skin care composition has the appearance of a solid. However, as shear stress is increased, the skin care composition can be made to flow as a fluid.) This property ensures the stability of the skin care composition under static conditions (e.g., under condition where the product is in storage and/or transportation) and maximize the availability of the skin care composition transferred to the skin for the skin effects under mechanical shear (e.g., under condition where the product is worn by the wearer and shear stress is applied to the skin care composition).

The skin care composition may have viscosity of more than about $10^5$ Poise under shear stress of less than about $3X10^4$ dynes/cm$^2$, and viscosity of less than about $10^2$ Poise under shear stress of more than about $10^6$ dynes/cm$^2$, at 40°C. Preferably, the skin care composition may have viscosity of more than about $10^6$ Poise under shear stress of less than about $3X10^4$ dynes/cm$^2$, and viscosity of less than about $10^1$ Poise under shear stress of more than about $10^6$ dynes/cm$^2$, at 40°C. Herein, 40°C represents the temperature which the skin care composition potentially experiences while the article is in storage or transported or worn in contact with the skin. Shear stress of about $3X10^4$ dynes/cm$^2$ represents the maximum shear stress applied to the skin care composition while the article is in storage or transported. Shear stress of about $10^6$ dynes/cm$^2$ represents the shear stress applied to the skin care composition at least once while the article is worn. Viscosity of about $10^5$ Poise is the lowermost viscosity required for preventing the skin care composition from migrating in storage or transportation. Viscosity of more than about $10^6$ Poise is more preferable to prevent the skin care composition from migrating. Viscosity of about $10^2$ Poise is the uppermost viscosity required for obtaining the effective lubrication between the wearers skin and the body contacting surface of the article and transfer of the skin care composition onto the wearer's skin. Viscosity of less than about $10^1$ Poise is more preferable for effective lubrication and transfer of the skin care composition. Viscosity at various shear stress should be measured by available

Rheometers such as cone to plate or plate to plate type Rheometers (e.g. Rheometer SR-2000, Rheometrics Inc.), at 40°C.

FIG. 6 shows a graph of shear stress vs. viscosity of one example of preferred skin care composition. When the skin care composition has viscosity of more than about $10^5$ Poise under shear stress of about less than $3X10^4$ dynes/$cm^2$ at 40°C, the skin care composition has less tendency to migrate into the interior of the article in storage or transportation and has more tendency to remain on the body contacting surface of the article. When the skin care composition has viscosity of less than about $10^2$ Poise under shear stress of more than about $10^6$ dynes/$cm^2$ at 40°C, the skin care composition has more tendency to transfer to the wearer's skin. Thus, the skin care composition tends to remain stable on the body contacting surface of the article in storage or transportation, thereby requiring less initial amount of skin care composition disposed on the body contacting surface. Once the absorbent article is worn by the wearer, the skin care composition is exposed to higher shear stress than the shear stress in storage or transportation. Thereby, the skin care composition starts to flow and effective amount of the skin care composition is transferred to the wearer's skin. Herein, the term "effective amount of a skin care composition" refers to an amount of a particular composition which, when applied or migrated to ("disposed on") the body contacting surface, will be effective in delivering desirable skin effects mentioned above.

The skin care compositions also may have consistency of not more than about 300, preferably of not more than about 150, more preferably of not more than about 100, at 40°C. Herein "consistency" means resistance against deformation of skin care composition. When the skin care composition has consistency of not more than about 300 at 40°C, the skin care composition has more tendency to remain on the body contacting surface of the absorbent article. Consistency can be measured by Penetrometer described in ASTM D5, at 40°C. The Penetrometer which is used for measuring consistency is supplied by Petrolab Company, 874 Albany-Shaker Road, Latham, NY, under the designation of PNR-10.

The effective amount of composition disposed on the body contacting surface will depend, to a large extent, on the particular skin care composition used, a portion of the body contacting surface where the skin care composition is applied, and/or a product form (e.g., diaper, sanitary napkin, etc.). Nonetheless, the minimum quantity of the skin care composition disposed on at least a portion of the body contacting surface of absorbent articles will preferably be not less than about 0.05 mg/$in^2$ (0.0078 mg/$cm^2$), more preferably not less than about 0.1 mg/$in^2$ (0.016 mg/$cm^2$), still more preferably not less than about 0.5 mg/$in^2$ (0.078 mg/$cm^2$). The maximum quantity of the skin care composition disposed on at least a portion of the body contacting surface will preferably be not more than about 80 mg/$in^2$ (12 mg/$cm^2$), more preferably not more than about 25 mg/$in^2$ (3.9 mg/$cm^2$), still more preferably not more than about 5 mg/$in^2$ (0.8 mg/$cm^2$). These ranges are by way of illustration only and the skilled artisan will recognize that the nature of the composition will dictate the level that must be disposed thereon to achieve the desired skin effects, and that such levels are ascertainable by routine experimentation in light of the present disclosure. Particularly when the skin care composition such as that described in Example 1 is applied to the body contacting surface (i.e., the topsheet 38 of the main body portion 22 and the flap 24) of the sanitary napkin shown in FIG. 1, the quantity of the skin care composition disposed on the body contacting surface preferably ranges from about 0.05 mg/$in^2$ (0.0078 mg/$cm^2$) to about 25 mg/$in^2$ (3.9 mg/$cm^2$), more preferably from about 0.1 mg/$in^2$ (0.016 mg/$cm^2$) to about 10 mg/$in^2$ (1.5 mg/$cm^2$), still more preferably from about 0.5 mg/$in^2$ (0.078 mg/$cm^2$) to about 4 mg/$in^2$ (0.6 mg/$cm^2$).

The skin care compositions may comprise: (1) one or more emollient(s) (2) one or more immobilizing agent(s) to stabilize the emollient(s) (3) one or more skin care active ingredient(s), and (4) other optional components. Although the kind, grade and content of each component of the skin care compositions are arbitrary, the skin care composition including at least one skin care active ingredient preferably satisfies viscosity of more than about $10^5$ Poise under shear stress of less than about $3X10^4$ dynes/$cm^2$, and viscosity of less than about $10^2$ Poise under shear stress of more than about $10^6$ dynes/$cm^2$, at 40°C. Especially, when the skin care composition comprises emollients and immobilizing agents, the selected ranges of quantity of emollients and immobilizing agents help to achieve the above viscosity range. Namely, the skin care composition comprises from about 40 to about 90% of the emollient. Preferably, the skin care composition comprises from about 50 to about 85%, more preferably from about 60 to about 80%, of the emollient. The skin care composition comprises from about 10 to about 60% of the immobilizing agent. Preferably, the skin care composition comprises from about 15 to about 50%, more preferably from about 20 to about 40%, of the immobilizing agent.

1. Emollient

One key component of the skin care composition is one or more hydrophobic emollient(s). The emollients are selected from materials that soften, smoothen, coat, moisturize, lubricate or cleanses the skin. In general, an emollient simultaneously accomplishes several of these objectives such as smoothing, moisturizing and lubricating the skin. The emollient preferably has a viscoelastic consistency at 20°C. However, this is not essential as long as the skin care composition such as the combination of the emollients and the other components (e.g., immobilizing agents) satisfies the viscosity described above.

The emollients are also substantially free of water. Herein, substantially free of water means that water is not

intentionally added to the emollients. Addition of water to the emollients is not needed in preparing or using the skin care compositions. However, minor and or trace quantities of water in the emollient that are contained as the results of ambient humidity can be tolerated without adverse effect. Typically, the emollients may contain about 5% or less water, preferably about 1% or less water, most preferably about 0.5% or less water.

The emollients are preferably hydrophobic such that the sweat, feces, and/or the menses form larger contact angle against the emollient than against the skin and or the topsheet of the article. The hydrophobic emollient reduces wetting of the sweat, feces and/or the menses against the skin and or the topsheet of the article, thereby reducing sticky feeling on the wearer's skin.

The emollients include petroleum-based, fatty acid ester type, alkyl ethoxylate type, fatty acid ester ethoxylates, fatty alcohol type, polysiloxane type, or mixtures of them.

Petroleum-based emollients include petrolatum. Petrolatum is a mixture of aliphatic hydrocarbons having alkyl chain length of 16 or more and possesses inherently a jelly appearance and a jelly hand. Petrolatum is also sometimes categorized as a mixture of mineral oil and microcrystalline wax. Herein, mineral oil refers to a fraction of petrolatum having less viscous mixtures of hydrocarbons having alkyl chain length of from 16 to 24. Herein, microcrystalline wax refers to a fraction of petrolatum having more viscous mixtures of hydrocarbons having alkyl chain length of 25 or more. Petrolatum as the emollient provides the smoothness of the skin because of its jelly hand. Also, petrolatum as the emollient coats the skin or can be absorbed by skin, thereby resulting in moisturizing the skin.

Petrolatum contains heavy aliphatic hydrocarbons and light aliphatic hydrocarbons. The heavy aliphatic hydrocarbons and the light aliphatic hydrocarbons can be defined by alkyl chain length of alkanes in petrolatum including both normal chain and isomers. The light aliphatic hydrocarbons of petrolatum tends to migrate faster than the heavy aliphatic hydrocarbons of petrolatum because molecular weight and diameter of molecules determine the flow property (i.e., viscosity) and diffusion rate of aliphatic hydrocarbons. Therefore, it is preferable that the heavy aliphatic hydrocarbons presents more than the light aliphatic hydrocarbons in petrolatum. The amount of the heavy aliphatic hydrocarbons and the light aliphatic hydrocarbons is comparable by weight ((weight = (the number of the molecules) x (molecular weight of the molecules)). Reduction of the light aliphatic hydrocarbons contributes to reduce migration of petrolatum. However, too much heavy aliphatic hydrocarbons may render the skin care composition too hard to flow. Therefore, the balance between the amount of the heavy aliphatic hydrocarbons and the amount of the light aliphatic hydrocarbons should be made. Petrolatum preferably has the heavy aliphatic hydrocarbons having alkyl chain length of from 33-36 more than the light aliphatic hydrocarbons having alkyl chain length of from 23-26. In this case, the ratio of the heavy aliphatic hydrocarbons to the light aliphatic hydrocarbons is preferably between 2.0 : 1.0 and 1.0 : 1.0, more preferably between 1.5 : 1.0 and 1.0 : 1.0. More preferably, petrolatum has the heavy aliphatic hydrocarbons having alkyl chain length of from 30-36 more than the light aliphatic hydrocarbons having alkyl chain length of from 20-26. In this case, the ratio of the heavy aliphatic hydrocarbons to the light aliphatic hydrocarbons is preferably between 2.5 : 1.0 and 1.0 : 1.0, more preferably between 2.0 : 1.0 and 1.0 : 1.0. Yet preferably, petrolatum has the heavy aliphatic hydrocarbons having alkyl chain length of from 27-36 more than the light aliphatic hydrocarbons having alkyl chain length of from 17-26. In this case, the ratio of the heavy aliphatic hydrocarbons to the light aliphatic hydrocarbons is preferably between 3.5 : 1.0 and 1.5 : 1.0, more preferably between 3.0 : 1.0 and 2.0 : 1.0.

Suitable fatty acid esters include those derived from $C_{12}$-$C_{28}$ fatty acids, preferably $C_{16}$-$C_{24}$ saturated fatty acids, and short chain ($C_1$-$C_8$, preferably $C_1$-$C_3$) monohydric alcohols. Representative examples of such esters include methyl palmitate, methyl stearate, isopropyl laurate, isopropyl myristate, isopropyl palmitate, ethylhexyl palmitate and mixtures thereof. Suitable fatty acid esters can also be derived from esters of longer chain fatty alcohols ($C_{12}$-$C_{28}$, preferably $C_{12}$-$C_{16}$) and shorter chain fatty acids e.g. lactic acid such as lauryl lactate and cetyl lactate. Fatty acid esters as the emollients provide the smoothness of the skin. Also, fatty acid esters as the emollients coat the skin, thereby resulting in moisturizing the skin.

Suitable alkyl ethoxylates include $C_{12}$-$C_{22}$ fatty alcohol ethoxylates having an average degree of ethoxylation of from about 2 to about 30. Preferably, the fatty alcohol ethoxylate is selected from the group consisting of lauryl, cetyl, and stearyl ethoxylates, and mixtures thereof, having an average degree of ethoxylation ranging from about 2 to about 23. Representative examples of such alkyl ethoxylates include laureth-3 (a lauryl ethoxylate having an average degree of ethoxylation of 3), laureth-23 (a lauryl ethoxylate having an average degree of ethoxylation of 23), ceteth-10 (a cetyl alcohol ethoxylate having an average degree of ethoxylation of 10) and steareth-10 (a stearyl alcohol ethoxylate having an average degree of ethoxylation of 10). These alkyl ethoxylates as the emollients are typically used in combination with petrolatum as the emollient, at a weight ratio of alkyl ethoxylate emollient to petrolatum emollient of from about 1: 1 to about 1:5, preferably from about 1:2 to about 1:4.

Suitable fatty alcohols include $C_{12}$-$C_{24}$ fatty alcohols, preferably $C_{16}$-$C_{24}$ fatty alcohols, most preferable $C_{18}$-$C_{24}$ alcohols. Representative examples include cetyl alcohol, stearyl alcohol, behenyl alcohol and mixtures thereof. These fatty alcohols as the emollients are typically used in combination with petrolatum as the emollient, at a weight ratio of fatty alcohol emollient to petrolatum emollient of from about 1:1 to about 1:5, preferably from about 1:1 to about 1:2.

Other suitable types of emollients include polysiloxane compounds which sometimes are called silicone compounds or silicone polymer compounds instead. In general suitable polysiloxane compounds include those having monomeric siloxane units of the following structure:

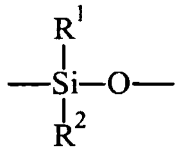

wherein, $R_1$ and $R_2$, for each independent siloxane monomeric unit can each independently be hydrogen or any alkyl, aryl, alkenyl, alkaryl, arakyl, cycloalkyl, halogenated hydrocarbon, or other radical. Any of such radicals can be substituted or unsubstituted. $R_1$ and $R_2$ radicals of any particular monomeric unit may differ from the corresponding functionalities of the next adjoining monomeric unit. Additionally, the polysiloxane can be either a straight chain, a branched chain or have a cyclic structure. The radicals $R_1$ and $R_2$ can additionally independently be other silaceous functionalities such as, but not limited to siloxanes, polysiloxanes, silanes, and polysilanes. The radicals $R_1$ and $R_2$ may contain any of a variety of organic functionalities including, for example, alcohol, carboxylic acid, phenyl, and amine functionalities.

Exemplary alkyl radicals are methyl, ethyl, propyl, butyl, pentyl, hexyl, octyl, decyl, octadecyl, and the like. Exemplary alkenyl radicals are vinyl, allyl, and the like. Exemplary aryl radicals are phenyl, diphenyl, naphthyl, and the like. Exemplary alkaryl radicals are toyl, xylyl, ethylphenyl, and the like. Exemplary aralkyl radicals are benzyl, alpha-phenylethyl, beta-phenylethyl, alpha-phenylbutyl, and the like. Exemplary cycloalkyl radicals are cyclobutyl, cyclopentyl, cyclohexyl, and the like. Exemplary halogenated hydrocarbon radicals are chloromethyl, bromoethyl, tetrafluorethyl, fluorethyl, trifluorethyl, trifluorotloyl, hexafluoroxylyl, and the like.

Viscosity of polysiloxanes useful may vary as widely as the viscosity of polysiloxanes in general vary, so long as the polysiloxane is flowable or can be made to be flowable for application to the article topsheet. This includes, but is not limited to, viscosity as low as 5 centistokes (at 37°C as measured by a glass viscometer) to about 20,000,000 centistokes. Preferably the polysiloxanes have a viscosity at 37°C ranging from about 5 to about 5,000 centistokes, more preferably from about 5 to about 2,000 centistokes, most preferably from about 100 to about 1000 centistokes. High viscosity polysiloxanes which themselves are resistant to flowing can be effectively deposited upon the topsheet of the article by such methods as, for example, emulsifying the polysiloxane in surfactant or providing the polysiloxane in solution with the aid of a solvent, such as hexane, listed for exemplary purposes only. Particular methods for applying polysiloxane emollients onto the topsheet of the article are discussed in more detail hereinafter.

Preferred polysiloxanes compounds are disclosed in U.S. Patent 5,059,282 (Ampulski et al), issued October 22, 1991. Particularly preferred polysiloxane compounds for use as emollients in the skin care compositions include phenyl-functional polymethylsiloxane compounds (e.g., Dow Corning 556 Cosmetic-Grade Fluid: polyphenylmethylsiloxane) and cetyl or stearyl functionalized dimethicones such as Dow 2502 and Dow 2503 polysiloxane fluids, respectively. In addition to such substitution with phenyl-functional or alkyl groups , effective substitution may be made with amino, carboxyl, hydroxyl, ether, polyether, aldehyde, ketone, amide, ester, and thiol groups. Of these effective substituent groups, the family of groups comprising phenyl, amino, alkyl; carboxyl, and hydroxyl groups are more preferred than the others; and phenyl-functional groups are most preferred.

Besides petrolatum emollient, fatty acid ester emollients, fatty acid ester ethoxylate emollients, alkyl ethoxylate emollients, fatty alcohol emollients, and polysiloxane compound emollients, the emollients can include minor amounts (e.g. up to about 10% of the total emollient) of other, conventional emollients. These other, conventional emollients include propylene glycol, glycerine, triethylene glycol, spermaceti or other waxes, fatty acids, and fatty alcohol ethers having from 12 to 28 carbon atoms in their fatty chain, such as stearic acid, propoxylated fatty alcohols; glycerides, acetoglycerides, and ethoxylated glycerides of $C_{12}$-$C_{28}$ fatty acids; other fatty esters of poly hydroxy alcohols; lanolin and its derivatives. These other emollients should be included in a manner such that the solid or semisolid characteristics of the skin care composition are maintained.

The amount of emollient that can be included in the skin care composition will depend on a variety of factors, including the particular emollient involved, the desirable benefits, the other components in the skin care composition and like factors.

2. Immobilizing Agent

Another key component of skin care compositions is immobilizing agents. Immobilizing agents are capable of thickening emollients of skin care compositions. Immobilizing agents may be soluble in emollients or insoluble in emollients. Without wishing to be bound by the theory, it is believed that immobilizing agents form microscopic networks in the skin care composition (e.g., emollient) to thicken the skin care composition. Herein, "microscopic network" refers to

the network whose units are molecules of immobilizing agents being soluble in emollients, or particles of immobilizing agents being insoluble in emollients. Particularly, "microscopic network" of immobilizing agents being soluble in emollients is sometimes referred to as "liquid crystal". In the microscopic network, molecules or particles form flexible/weak connection among molecules or particles.

The immobilizing agent should be homogeneously dispersed in the skin care composition such that less amount of immobilizing agent is required to impart expected thickening. Less amount of immobilizing agent contained in the skin care composition allows more amount of emollient, which is one of the actives for expected skin effects, in the skin care composition. The immobilizing agent homogeneously dispersed in the skin care composition also enables to control variability of the quality of the skin care composition. Preferably, the immobilizing agent has sufficient solubility or dispersability in the emollient. In most cases and preferred cases, the emollients are hydrophobic. In this case the immobilizing agents should be also hydrophobic so that the emollient and the immobilizing agent can achieve a desired homogeneous mixture. The immobilizing agents and the emollients are sometimes compounded while both of them are molten.

The immobilizing agents being soluble in emollients can comprise a component selected from the group consisting of $C_{14}$-$C_{24}$ fatty alcohols, $C_{12}$-$C_{24}$ fatty acids, and $C_{12}$-$C_{24}$ fatty alcohol ethoxylates having an average degree of ethoxylation ranging from 2 to about 30, and mixtures thereof. Preferred immobilizing agents include $C_{16}$-$C_{24}$ fatty alcohols, more preferably $C_{18}$-$C_{24}$ fatty alcohols. Representative examples include stearyl alcohol, arachidyl alcohol, behenyl alcohol, lignoceryl alcohol, and mixtures thereof. Herein, "stearyl alcohol" refers to a mixtured fatty alcohol containing $C_{18}$ fatty alcohol as a major component. Herein, "arachidyl alcohol" refers to a mixtured fatty alcohol containing $C_{20}$ fatty alcohol as a major component. Herein, "behenyl alcohol" refers to a mixtured fatty alcohol containing $C_{22}$ fatty alcohol as a major component. Herein, "lignocaryl alcohol" refers to a mixtured fatty alcohol containing $C_{24}$ fatty alcohol as a major component. Preferably, the immobilizing agent comprises behenyl alcohol. Behenyl alcohol as the immobilizing agent preferably comprises $C_{22}$ fatty alcohol as a major component and a minor quantity of $C_{24}$ fatty alcohol. Behenyl alcohol may also comprise $C_{18}$ fatty alcohol and $C_{20}$ fatty alcohol. By containing a minor quantity of $C_{24}$ fatty alcohol in behenyl alcohol, behenyl alcohol provides the emollients with more stability and more efficiently immobilizes the emollients of the skin care composition. When behenyl alcohol comprises $C_{18}$ fatty alcohol, $C_{20}$ fatty alcohol, $C_{22}$ fatty alcohol, and $C_{24}$ fatty alcohol, the ratio of each component is preferably as follows: from about 50 % to about 99.99 % of $C_{22}$ fatty alcohol, more preferably from about 63 % to about 84.9 % of $C_{22}$ fatty alcohol; from about 0.01 % to about 3 % of $C_{24}$ fatty alcohol, more preferably from about 0.1 % to about 2 % of $C_{24}$ fatty alcohol; from 0 % to about 27 % of $C_{20}$ fatty alcohol, more preferably from about 10 % to about 20 % of $C_{20}$ fatty alcohol; from 0 % to about 20 % of $C_{18}$ fatty alcohol, more preferably from about 5 % to about 15 % of $C_{18}$ fatty alcohol. One of preferable behenyl alcohols as the immobilizing agent is Lanette 22 available from Henkel Corp. Cospha, 300 Brookside Avenue,Ambler, PA 19002.

Other preferred immobilizing agents include $C_{16}$-$C_{24}$ fatty acids, more preferably $C_{18}$-$C_{24}$ fatty acids selected from the group consisting of stearic acid, behenic acid and mixtures thereof. Behenic acid is most preferred. Still other preferred immobilizing agents include $C_{16}$-$C_{24}$ fatty alcohol ethoxylates having an average degree of ethoxylation ranging from about 5 to about 20. Preferably, the fatty alcohols, fatty acids and fatty alcohols are linear and do not contain branched isomers.

Importantly, these preferred immobilizing agents such as the $C_{18}$-$C_{24}$ fatty alcohols are assumed to provide the microscopic network in the skin care composition, thereby resulting in the skin care composition which is far thicker than only the emollients which are included in the skin care composition. These preferred immobilizing agents such as $C_{18}$-$C_{24}$ fatty alcohols are soluble enough to form homogeneous mixtures of the emollients and the immobilizing agents.

Other types of immobilizing agents being soluble in emollients can be used either alone or in combination with the fatty alcohols, fatty acids, and fatty alcohol ethoxylates described above. Examples of these other types of immobilizing agents includes polyhydroxy fatty acid esters, polyhydroxy fatty acid amides, and mixtures thereof. Preferred esters and amides will have three or more free hydroxy groups on the polyhydroxy moiety and are typically nonionic in character. Because of the possible skin sensitivity of those using article topsheets to which the skin care composition is applied, these esters and amides should also be relatively mild and non-irritating to the skin.

Suitable polyhydroxy fatty acid esters will have the formula:

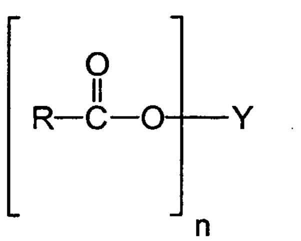

wherein R is a $C_5$-$C_{31}$ hydrocarbyl group, preferably straight chain $C_7$-$C_{19}$ alkyl or alkenyl, more preferably straight chain $C_9$-$C_{17}$ alkyl or alkenyl, most preferably straight chain $C_{11}$-$C_{17}$ alkyl or alkenyl, or mixture thereof; Y is a polyhydroxyhydrocarbyl moiety having a hydrocarbyl chain with at least 2 free hydroxyls directly connected to the chain; and n is at least 1. Suitable Y groups can be derived from polyols such as glycerol, pentaerythritol; sugars such as raffinose, maltodextrose, galactose, sucrose, glucose, xylose, fructose, maltose, lactose, mannose and erythrose; sugar alcohols such as erythritol, xylitol, malitol, mannitol and sorbitol; and anhydrides of sugar alcohols such as sorbitan.

One class of suitable polyhydroxy fatty acid esters comprises certain sorbitan esters, preferably the sorbitan esters of $C_{16}$-$C_{22}$ saturated fatty acids. Because of the manner in which they are typically manufactured, these sorbitan esters usually comprise mixtures of mono-, di-, tri-, etc. esters. Representative examples of suitable sorbitan esters include sorbitan palmitates (e.g., SPAN 40), sorbitan stearates (e.g., SPAN 60), and sorbitan behenates, that comprise one or more of the mono-, di- and tri-ester versions of these sorbitan esters, e.g., sorbitan mono-, di- and tri-palmitate, sorbitan mono-, di- and tri-stearate, sorbitan mono-, di and tri-behenate, as well as mixed tallow fatty acid sorbitan mono-, di- and tri-esters. Mixtures of different sorbitan esters can also be used, such as sorbitan palmitates with sorbitan stearates. Particularly preferred sorbitan esters are the sorbitan stearates, typically as a mixture of mono-, di- and tri-esters (plus some tetraester) such as SPAN 60, and sorbitan stearates sold under the trade name GLYCOMUL-S by Lonza, Inc. Although these sorbitan esters typically contain mixtures of mono-, di- and tri-esters, plus some tetraester, the mono- and di-esters are usually the predominant species in these mixtures.

Another class of suitable polyhydroxy fatty acid esters comprises certain glyceryl monoesters, preferably glyceryl monoesters of $C_{16}$-$C_{24}$ saturated fatty acids such as glyceryl monostearate, glyceryl monopalmitate, and glyceryl monobehenate. Again, like the sorbitan esters, glyceryl monoester mixtures will typically contain some di- and triester. However, such mixtures should contain predominantly the glyceryl monoester species.

Another class of suitable polyhydroxy fatty acid esters comprises certain sucrose fatty acid esters, preferably the $C_{12}$-$C_{24}$ saturated fatty acid esters of sucrose. Sucrose monoesters and diesters are particularly preferred and include sucrose mono- and distearate and sucrose mono- and di- laurate.

Suitable polyhydroxy fatty acid amides will have the formula:

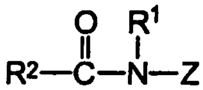

wherein $R^1$ is H, $C_1$-$C_4$ hydrocarbyl, 2-hydroxyethyl, 2-hydroxypropyl, methoxyethyl, methoxypropyl or a mixture thereof, preferably $C_1$-$C_4$ alkyl, methoxyethyl or methoxypropyl, more preferably $C_1$ or $C_2$ alkyl or methoxypropyl , most preferably $C_1$ alkyl (i.e., methyl) or methoxypropyl; and $R^2$ is a $C_5$-$C_{31}$ hydrocarbyl group, preferably straight chain $C_7$-$C_{19}$ alkyl or alkenyl, more preferably straight chain $C_9$-$C_{17}$ alkyl or alkenyl, most preferably straight chain $C_{11}$-$C_{17}$ alkyl or alkenyl, or mixture thereof; and Z is a polyhydroxyhydrocarbyl moiety having a linear hydrocarbyl chain with at least 3 hydroxyls directly connected to the chain. See U.S. patent 5,174, 927 (Honsa), issued December 29, 1992 which discloses these polyhydroxy fatty acid amides, as well as their preparation.

The Z moiety preferably will be derived from a reducing sugar in a reductive amination reaction; most preferably glycityl. Suitable reducing sugars include glucose, fructose, maltose, lactose, galactose, mannose, and xylose. High dextrose corn syrup, high fructose corn syrup, and high maltose corn syrup can be utilized, as well as the individual sugars listed above. These corn syrups can yield mixtures of sugar components for the Z moiety.

The Z moiety preferably will be selected from the group consisting of $-CH_2-(CHOH)_n-CH_2OH$, $-CH(CH_2OH)-[(CHOH)_{n-1}]-CH_2OH$, $-CH_2OH-CH_2(CHOH)_2(CHOR^3)(CHOH)-CH_2OH$, where n is an integer from 3 to 5, and $R^3$ is H or a cyclic or aliphatic monosaccharide. Most preferred are the glycityls where n is 4, particularly $-CH_2-(CHOH)_4-CH_2OH$.

In the above formula, $R^1$ can be, for example, N-methyl, N-ethyl, N-propyl, N-isopropyl, N-butyl, N-2-hydroxyethyl, N-methoxypropyl or N-2-hydroxypropyl,. $R^2$ can be selected to provide, for example, cocamides, stearamides, oleamides, lauramides, myristamides, capricamides, palmitamides, tallowamides, etc. The Z moiety can be 1-deoxyglucityl, 2-deoxyfructityl, 1-deoxymaltityl, 1-deoxylactityl, 1-deoxygalactityl, 1-deoxymannityl, 1-deoxymaltotriotityl, etc.

The most preferred polyhydroxy fatty acid amides have the general formula:

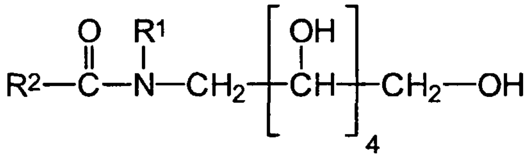

wherein $R^1$ is methyl or methoxypropyl; $R^2$ is a $C_{11}$-$C_{17}$ straight-chain alkyl or alkenyl group. These include N-lauryl-N-methyl glucamide, N-lauryl-N-methoxypropyl glucamide, N-cocoyl-N-methyl glucamide, N-cocoyl-N-methoxypropyl glucamide, N-palmityl-N-methoxypropyl glucamide, N-tallowyl-N-methyl glucamide, or N-tallowyl-N-methoxypropyl glucamide.

As previously noted, some of the immobilizing agents require an emulsifier for solubilization in the emollient. This is particularly the case for certain of the glucamides such as the N-alkyl-N-methoxypropyl glucamides having HLB values of at least about 7. Suitable emulsifiers will typically include those having HLB values below about 7. In this regard, the sorbitan esters previously described, such as the sorbitan stearates, having HLB values of about 4.9 or less have been found useful in solubilizing these glucamide immobilizing agents in petrolatum. Other suitable emulsifiers include steareth-2 (polyethylene glycol ethers of stearyl alcohol that conform to the formula $CH_3(CH_2)_{17}(OCH_2CH_2)_nOH$, where n has an average value of 2), sorbitan tristearate, isosorbide laurate, and glyceryl monostearate. The emulsifier can be included in an amount sufficient to solubilize the immobilizing agent in the emollient such that a substantially homogeneous mixture is obtained. For example, an approximately 1:1 mixture of N-cocoyl-N-methyl glucamide and petrolatum that will normally not melt into a single phase mixture, will melt into a single phase mixture upon the addition of 20% of a 1:1 mixture of Steareth-2 and sorbitan tristearate as the emulsifier.

Other types of ingredients being soluble in emollients that can be used as immobilizing agents, either alone, or in combination with the above-mentioned immobilizing agents, include waxes such as carnauba wax, beeswax, candelilla wax, paraffin wax, microcrystalline wax, castol wax, ceresin, esparto, ouricuri, rezowax, polyethylene wax, and other known waxes. Preferably the wax is a microcrystalline wax. An example of a particularly preferred microcrystalline wax is Multiwax W-835, Witco Corporation, One American Lane, Greenwich, CT06831-2559.

The immobilizing agents being insoluble in emollients are particulate thickeners. Herein "particulate thickener" means the immobilizing agent being in the form of small, finely divided particles. The particulate thickener is able to enhance smooth feeling or lubricant effect of the skin care composition because of its particle structure. For example, fumed silica is so spherical like a "ball" that it can act as a bearing, when shear force is applied, resulting in a lubricant effect. Bentonite or hectorite consists of a plurality of thin layers which can slide easily thus resulting in smooth feeling. The average diameter or the corresponding average diameter may be less than about 100 microns, preferably less than about 20 microns, more preferably less than about 10 microns. When the particle has a different shape from a sphere, the corresponding diameter of the non-spherical particle can be represented by a diameter of a sphere having the same volume as the non-spherical particle. The corresponding diameter can be measured by Laser Particle Analyzer supplied by Honeywell Inc., Honeywell Plaza, Minneapolis, MN, under the designation of Microtrac X-100.

The particulate thickener is essentially insoluble in the emollient but can be dispersed forming the microscopic network therein and provide a solid structure for the emollient. The particulate thickener can be selected from the group consisting of silica, treated silica, polymethacrylate polymers, polymethacrylate and styrene copolymers, calicum silicate, treated calcium silicate, treated bentonite, treated hectorite, and mixtures thereof. A preferred particulate thickener for use herein is fumed silica. More preferred for use herein is surface-treated fumed silica. Even more preferred is a fumed silica selected from the group consisting of polyalkylsiloxane treated fumed silica, trialkylsilanized fumed silica, dialkyldisilanized fumed silica, and mixtures thereof. Most preferred is a fumed silica selected from the group consisting of polydimethylsiloxane treated fumed silica, trimethylsilanized fumed silica, dimethyldisilanized fumed silica, and mixtures thereof.

Silica is also known as silicon dioxide or silicic anhydride, which can be represented by the chemical formula SiO2. A variety of different types of silicas which are useful herein, are known fumed or arced silica, precipitated silica, silica gel, amorphous silica, and silica sols and colloids. Fumed silica, which is also known as arced silica, is produced by the vapor phase hydrolysis of silicon tetrachloride in a hydrogen oxygen flame. Without being limited by theory, it is believed that the combustion process creates silicon dioxide molecules which condense to form particles. The particles collide, attach and sinter together. The result of this process is a three dimensional branched chain aggregate. Once the aggregate cools below the fusion point of silica, which is about 1710° C., further collisions result in mechanical entanglement of the chains to form agglomerates. Precipitated silicas and silica gels are generally made in aqueous solution. Amorphous silicas are generally naturally occurring microcrystalline forms of the material. Silica sols and colloids are dispersions of amorphous silica in an aqueous solution

The fumed silica and treated fumed silica preferably have a mean particle size for the agglomerates, i.e., a

mean agglomerate particle size, of from about 0.1 microns to about 100 microns, preferably from about 1 micron to about 50 microns, and more preferably from about 10 microns to about 30 microns. The agglomerates are composed of aggregates which having a mean particle size, i.e., a mean aggregate particle size, from about 0.01 microns to about 15 microns, preferably from about 0.05 microns to about 10 microns, more preferably from about 0.1 microns to about 5 microns, and most preferably from about 0.2 microns to about 0.3 microns.

The fumed silica agglomerates typically have active hydroxyl groups. It is desirable to treat these fumed silicas to render the hydroxyl groups less reactive especially when the emollient in the skin care composition is hydrophobic. A useful method of treatment is to coat the fumed silica with a nonpolar organic compound to render the active hydroxyl groups less reactive. Preferred organic compounds for treatment include polyalkylsiloxanes, with polydimethylsiloxanes being most preferred. A commercially available polydimethylsiloxane treated fumed silicas useful herein are sold under the trade name CAB-O-Sil® TS-720 by Cabot Corporation, Tuscola, IL, or Aerosil R972 by Degussa AG, Germany, both of which have a surface area of around 120 $m^2$/g and a bulk density of 50 g/L. Another useful method of treatment is to chemically react the hydroxyl groups of the fumed silica with a silanizing agent, e.g., diemthyldichlorosiliane or hexamethyldisilizane. In these chemically treated silicas, the free hydroxyl groups of the silica are replaced with an oxygen-silicon bond of the silanizing agent. A commercially available trimethyl silanized fumed silica is sold under the trade name CAB-0-Sil® TS-530, by Cabot Corporation, Tuscola, IL, which has a surface area of about 220 $m^2$/g and a bulk density of 50 g/L. A commercially available dimethyldisilanized fumed silica is sold under the trade name CAB-O-SIL® TS-610, by Cabot Corporation, Tuscola, IL, which has a surface area of about 120 $m^2$/g and a bulk density of 50 g/L.

The particulate thickeners also useful herein are polymethacrylate polymers and polymethacrylate and styrene copolymers. These materials are swellable polymers which are useful for absorbing liquid compositions to provide a thickening or gelling effect on the liquid. The polymethacrylate polymers are homopolymers of methacrylic acid esters, preferably the methyl or ethyl esters, which are optionally crosslinked with any of the common crosslinking agents. The polymethacrylate and styrene copolymers are copolymers of methacrylic acid esters, preferably the methyl or ethyl esters, withstyrene, which are optionally crosslinked with any of the common crosslinking agents. The crosslinking agent is most typically a material containing two or more unsaturated functional groups. The crosslinking agent is reacted with the monomer units of the polymer or copolymer and is incorporated into the polymer thereby forming links or covalent bonds between two or more individual polymer chains or between two or more sections of the same polymer chain. Non limiting examples of suitable crosslinking agents include those selected from the group consisting of methylenebisacrylamides, diallyldialkyl ammonium halides, polyalkenyl polyethers of polyhydric alcohols, allyl acrylates, vinyloxyalkylacrylates, and polyfunctional vinylidenes. Specific examples of crosslinking agents useful herein include those selected from the group consisting of methylenebisacrylamide, ethylene glycol di-(meth)acrylate, di-(meth)acrylamide, cyanomethylacrylate, vinyloxyethylacrylate, vinyloxyethylmethacrylate, allyl pentaerythritol, trimethylolpropane diallylether, allyl sucrose, butadiene, isoprene, divinyl benzene, divinyl naphthalene, ethyl vinyl ether, methyl vinyl ether, and allyl acrylate. Other crosslinkers include formaldehyde and glyoxal. A particularly useful crosslinked polymethacrylate polymer is sold under the trade name Polytrap®6603, by Dow Coming Corp., Midland, Mich.

The particulate thickeners also useful herein are treated bentonites and treated hectorites. Bentonite is a colloidal aluminum silicate clay. Hectorite is a clay containing sodium, magnesium, lithium silicon, oxygen, hydrogen, and fluorine. Useful herein especially when the emollient in the skin care composition is hydrophobic are bentonites and hectorites that have been treated with various organic compounds to render the clays less polar. Herein "treated" means that these materials have been coated with the organic compound. Non limiting examples of treated bentones include stearalkonium hectorite; quaternium-18 bentonite; quaternium-18 hectorite; castor oil and stearalkonium hectorite and propylene carbonate; isopropyl myristate and stearalkonium hectorie and propylene carbonate; isododecane and quaternium-18 hectorite and propylene carbonate; lanolin oil and isopropyl palmitate and stearalkonium hectorite and propylene carbonate and propylparaben; propylene glycol dicaprylate/dicaprate and stearalkonium hectorite and propylene carbonate; mineral oil and quaternium-18 hectorite and propylene carbonate; mineral oil and quaternium-18 hectorite and SD alcohol 40; petroleum distillates and quaternium-18 hectorite and propylene carbonate; C12-15 alkyl bnezoate and stearalknoium hectorite and propylene carbonate; cyclomethicone and quaternium-18 hectorite and SD alcohol 40; cyclomethicone and quaternium-18 hectorite and propylene carbonate; and mixtures thereof.

The particulate thickeners also useful herein are calcium silicate and treated calcium silicate. Common forms of calcium silicate include CaSiO3, CaSiO4, and CaSiO5. These materials are also known as calcium salts of silicic acid. The calcium silicates can be treated with a wide variety of nonpolar organic compounds to make the materials more hydrophobic. Useful calcium silicates include the following commercially available materials: Hubersorb (Huber Corp., Harve de Grace, Md.), and Micro-Cel C, Micro-Cel E, and Micro-Cel T-38 (Celite Corp., Denver, Colo.)

Most of all the particulate thickeners above can be easily dispersed in the emollient in the skin care composition thanks to the surface treatments. Instead of the surface treatments or in combination of the treatments some emulsifiers are sometimes useful.

The amount of immobilizing agents that can be included in the skin care composition will depend on a variety of factors.

3. Skin Care Active Ingredient

Various skin care active ingredients may be incorporated in the skin care composition. These skin care actives preferably provides skin care benefits. These active ingredients include, but not limited to, skin care agents, proton donating agents, enzyme inhibitors, or mixtures thereof. The skin care composition allows to reduce the initial amount of the skin care active ingredients incorporated because of less migration in storage or transportation while delivering effective amount of skin care active ingredients to the skin in use.

The skin care agents may be added to deliver a therapeutic and/or skin protective benefit. It will be recognized that of the numerous materials useful in the skin care compositions delivered to skin, those that have been deemed safe and effective skin care agent and mixtures thereof are logical materials for use herein. Such materials include Category I actives as defined by the U.S. Food and Drug Administration's (FDA) Tentative Final Monograph on Skin Protectant Drug Products for Over-the-Counter Human Use (21 C.F.R. § 347), which presently include: allantoin, aluminum hydroxide gel, calamine, cocoa butter, dimethicone, cod liver oil (in combination), glycerine, kaolin, petrolatum, lanolin, mineral oil, shark liver oil, white petrolatum, talc, topical starch, zinc acetate, zinc carbonate, zinc oxide, and the like. Other potentially useful materials are Category III actives as defined by the U.S. Food and Drug Administration's Tentative Final Monograph on Skin Protectant Drug Products for Over-the-Counter Human Use (21 C.F.R. § 347), which presently include: live yeast cell derivatives, aldioxa, aluminum acetate, microporous cellulose, cholecalciferol, colloidal oatmeal, cysteine hydrochloride, dexpanthenol, Peruvean balsam oil, protein hydrolysates, racemic methionine, sodium bicarbonate, Vitamin A, buffered mixture of cation and anion exchange resins, corn starch, trolamine, and the like. Further, other potential materials are Category II actives as defined by the U.S. Food and Drug Administration's Tentative Final Monograph on Skin Protectant Drug Products for Over-the-Counter Human Use (21 C.F.R. § 347), which include: bizmuth subnitrate, boric acid, ferric chloride, polyvinyl pyrrolidone - vinyl acetate copolymers, sulfur, tannic acid, and the like. The skin care agent may be selected from these materials and mixtures thereof. As mentioned above, the materials for use should be safe.

The skin care composition may include between about 0.001 % and about 50 % of the skin care agent. The concentration range of the skin care agents in the skin care composition varies from material to material. Below table shows preferred skin care agents for use and its concentration ranges in the skin care composition.

| | Possible concentration range (%) | Preferred concentration range (%) |
|---|---|---|
| allantoin | 0.2 - 5 | 0.5 - 2 |
| aluminum hydroxide gel | 0.1 - 10 | 0.15 - 5 |
| calamine | 0.2 - 40 | 1 - 25 |
| cocoa butter | 30 - 50 | 40 - 50 |
| dimethicone | 0.2 - 40 | 1 - 30 |
| glycerine | 5 - 50 | 20 - 45 |
| kaolin | 2 - 30 | 4 - 20 |
| petrolatum | 30 - 50 | 40 - 50 |
| shark liver oil | 0.1 - 10 | 1 - 5 |
| white petrolatum | 30 - 50 | 40 - 50 |
| zinc acetate | 0.05 - 10 | 0.1 - 2 |
| zinc carbonate | 0.05 - 10 | 0.2 - 2 |
| zinc oxide | 0.1 - 25 | 0.3 - 8 |

It should be understood that the skin care composition may include the materials listed above beyond its range. For example, when the skin care composition comprises 40 % of petrolatum as an emollient and 50 % of petrolatum as an skin care agent (another 10 % may be immobilizing agent), 90 % of the skin care composition may be petrolatum.

Many of the FDA monographed skin care agents are currently utilized in commercially available skin care products, such as A and D® Ointment, Vaseline® Petroleum Jelly, Desitin® Diaper Rash Ointment and Daily Care® ointment, Gold Bond ® Medicated Baby Powder, Aquaphor® Healing Ointment, Baby Magic® Baby Lotion, Johnson's Ultra Sensitive® Baby Cream. Those skin case ingredients and/or products may be incorporated to create treated articles.

The proton donating actives may be added to help maintain a wearer's skin at its natural acidic pH. For example

such proton donating actives can be effective in neutralizing any high pH (i.e. >7) components of bodily exudates. Chemically suitable proton donating actives are effective in helping maintain skin pH in at least a slightly acidic condition. A non limiting and exemplary listing of proton donating actives includes: monomeric organic acids; acid salts of organic or inorganic acids; and polymeric organic acids and salts thereof. Certain combinations of an acid and a salt thereof, commonly known as buffers, are also suitable as long as an aqueous solution of the acid/salt combination has a pH less than 7. A suitable acid or acid salt should have at least one pKa between about 2.0 and about 6.5. The preferred range of pKa values for suitable proton donating actives is between about 2.5 and about 5.0. Preferred proton donating actives include pharmaceutically acceptable monomeric and polymeric organic acids.

Exemplary monomeric organic acids suitable for use include: citric, malic, adipic, glutaric, lactic, sorbic, salicylic, tartaric, maleic, fumaric, malonic, glycolic, and succinic acids.

Exemplary organic polymeric acids include: acidic vinyl polymers, for example, homopolymers of unsaturated carboxylic acid and anhydride monomers such as acrylic acid itself, methacrylic acid, α-chloroacrylic acid, α-cyanoacrylic acid, β-methylacrylic acid (crotonic acid), α-phenylacrylic acid, β-acryloxypropionic acid, sorbic acid, α-chlorosorbic acid, angelic acid, cinnamic acid, p-chlorocinnamic acid, β-stearylacrylic acid, itaconic acid, citraconic acid, mesaconic acid, glutaconic acid, aconitic acid, maleic acid, fumaric acid, tricarboxyethylene, and maleic anhydride, copolymers of unsaturated monomeric acids with suitable co-monomers, and partially neutralized salts of such polymers; acidic cellulose derivatives, such as carboxymethyl cellulose at least partially wherein the cellulose derivatives are at least partially protonated, cellulose phosphate, and oxidized cellulose; and cation exchange resins wherein the cation exchange resin is at least partially protonated.

Exemplary inorganic acid salts include alkali metal monohydrogen phosphates, blends of alkali metal monohydrogen and dihydrogen phosphate salts, alkali metal monohydrogen pyrophosphate salts, and blends of alkali metal monohydrogen and dihydrogen pyrophosphate salts.

Materials which can decompose in the environment adjacent to a wearer's skin into a proton donating active are also suitable for use. For example, esterase enzymes in feces (e.g. fecal lipases) can hydrolyze certain esters to provide a proton donating active. Suitable proton donating actives of this type have the formula:

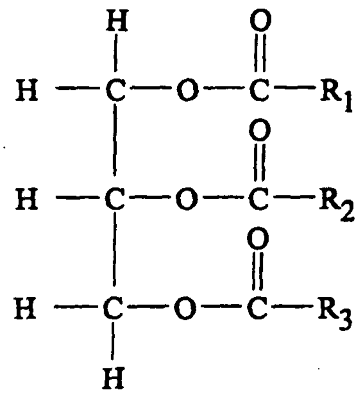

wherein $R_1$, $R_2$ and $R_3$ are independently hydrogen, an alkyl group, an alkenyl group, or a hydroxyalkyl group with from 1 to 4 carbon atoms. An exemplary ester of this type is triacetin.

In order that the proton donating active be effective in helping maintain skin at an acidic pH the proton donating active should be provided at a level of at least 0.01 % of the skin care composition; typically at least about 0.5%; preferably at least about 3%. Such suitable proton donating actives may be used as a component of the skin care compositions at a level of between about 0.01% and about 40%, depending on the specific proton donating active chosen. Preferably, the proton donating actives are used at a level of between about 0.5% and about 20% of the skin care composition. In particularly preferred embodiments, the proton donating actives are provided at a level of between about 3% and about 7%.

These and other features of proton donating actives are disclosed in U.S. Patent Application Serial No. 09/041509 entitled "Proton Donating Actives in Absorbent Articles" filed in the name of McOsker, et al. on March 12, 1998.

The inhibitors against enzymes may be added to be available at the skin/urine, skin/feces, skin/menses interfaces to inhibit enzymatic activity on the skin and to reduce or prevent the occurrence of inflammation. Inhibitors of enzyme activity are well known and are typically classified as competitive inhibitors (which compete with the substrate for binding at the active site on the enzyme) and non-competitive inhibitors (which bind to a site other than the active site to inactivate the enzyme). Many enzymes, such as metalloproteases, are inhibited by substances that bind with a metal group on the enzyme. Chelating agents are effective inhibitors of other enzymes that require the presence of metal ions, such as the ions of calcium, cobalt, copper, magnesium, manganese, sodium, potassium, or zinc, for activation.

Since enzymes are proteins, antibodies raised against specific enzymes are also effective enzyme inhibitors.

Enzyme inhibitors useful in the absorbent articles described herein will typically have an $IC_{50}$ value of not more than about 500 μM, more typically not more than about 250 μM, and still more typically not more than about 100 μM. As used herein, the term "$IC_{50}$"means the inhibitory concentration (e.g., a micromolar concentration, μM) of a substance (inhibitor) which reduces the rate of substrate cleavage by an enzyme by 50%. The $IC_{50}$ is calculated according to the equation $IC_{50}$ =[I]/[(v/$v_i$)-1], where [I] is the inhibitor concentration tested, v is the rate of substrate cleavage in the absence of the inhibitor and $v_i$ is the rate of substrate cleavage in the presence of the inhibitor. The $IC_{50}$ of an enzyme inhibitor may be measured by a Purified Enzyme method or by a Fecal Enzyme method which is described in U.S. Patent Application Serial No. 09/041266 entitled "Disposable Absorbent Article Having A Skin Care Composition Containing An Enzyme Inhibitor" filed in the name of Roe, et al. on March 12, 1998. It will be understood that certain enzyme inhibitors (e.g., EDTA) will have higher $IC_{50}$ values but will still be useful in the absorbent articles described herein. For materials for which the molecular weight cannot be determined, such materials will typically reduce enzyme activity by at least 50% at a concentration in the skin care composition of not more than about 5 percent by weight.

Without limitation, any type of enzyme inhibitor may be employed in the skin care compositions transferable to the wearer's skin, including any naturally occurring inhibitor of plant, microbial and/or animal origin (including human) and synthetically manufactured chemical inhibitor. The enzyme inhibitors may be hydrophilic or hydophobic in nature and may thus be water soluble or soluble in a hydrophobic vehicle. The enzyme inhibitors are preferably present in the skin care composition in a concentration of about 0.001 % to about 50% by weight, typically about 0.01 % to about 25%, more typically about 0.1% to about 10%, and most typically about 0.1 % to about 5%. Because of the variety of enzyme inhibitors employed, the effective concentration of each inhibitor must be separately determined, as known to those skilled in the art.

The enzyme inhibitors may be employed singly or as a mixture of enzyme inhibitors such as a "cocktail" of inhibitors in a single absorbent article. Moreover, different enzyme inhibitors may be employed in skin care compositions in different locations in a single absorbent article.

Because of the wide diversity of enzymes present in body exudates, it is reasonably predictable that materials such as those described below which inhibit certain classes of enzymes (e.g., proteases) may also inhibit enzymes which cleave substrates other than those specified (e.g., proteins and peptides). Hence, inhibitors which inhibit proteases may also inhibit lipases and other esterases, amylases and/or ureases and vice versa.

Inhibitors of enzymes and/or coenzymes most frequently found in body exudates are preferred in the skin care compositions. Thus, the enzyme inhibitors are preferably inhibitors of proteolytic enzymes such as trypsin, chymotrypsin, aminopeptidase and elastase; lipases; bile salts; amylases; and/or ureases.

Exemplary suitable inhibitors of proteases for use that are believed to inhibit the type of protease indicated in parentheses include, but are not limited to, soybean trypsin inhibitor and other plant-derived trypsin inhibitors such as lima bean protease inhibitor, corn protease inhibitor and the like; Bowman-Birk inhibitor (serine, trypsin-like protease inhibitor); pancreatic trypsin inhibitor such as bovine pancreatic basic trypsin inhibitor and other animal-derived pancreatic trypsin inhibitors; egg white trypsin inhibitor (serine, trypsin-like protease inhibitor); ovomucoids containing ovoinhibitors such as from chicken or turkey egg white (trypsin and chymotrypsin inhibitors); chymostatin (serine, chymotrypsin-like protease inhibitor); aprotinin (serine protease inhibitor); leupeptin and its analogs such as propionyl-leupeptin, N-α-*t*-BOC-deacetylleupeptin (serine and cysteine protease inhibitor); bestatin and its analogs such as epibestatin and nitrobestatin (aminopeptidase metalloprotease inhibitor); amastatin and its analogs such as epiamastatin (aminopeptidase inhibitor); antipain (trypsin inhibitor); antithrombin III (serine protease inhibitor); hirudin (thrombin-like serine protease inhibitor); cystatin (egg white cysteine protease inhibitor); E-64 (trans-epoxysuccinyl-$_L$-leucylamido-(4-guanidino)-butane) and its analogs (cysteine protease inhibitor); $\alpha_2$-macroglobulin (universal endoprotease inhibitor); $\alpha_1$-antitrypsin (trypsin inhibitor); pepstatin and its analogs such as acetyl pepstatin, pepstatin A, Nle-Sta-Ala-Sta (aspartyl protease inhibitor); apstatin (aminopeptidase P inhibitor); (2R)-2-mercaptomethyl-4-methylpentanoyl-b-(2-naphthyl)-Ala-Ala amide (matrix metalloprotease inhibitor); (2R)-2-mercaptomethyl-4-methylpentanoyl-Phe-Ala amide (matrix metalloprotease inhibitor); N-acetyl-Leu-Leu-methioninal (calpain inhibitor); N-acetyl-Leu-Leu-norleucinal (calpain inhibitor); p-aminobenzyol-Gly-Pro-$_D$-Leu-$_D$-Ala hydroxamic acid (matrix metalloprotease inhibitor); 2(R)-[N-(4-methoxyphenylsulfonyl)-N-(3-pyridylmethyl)amino]-3-methylbutano-hydroxamic acid (metalloprotease inhibitor); L-1-chloro-3-[4-tosylamido]-7-amino-2-heptanone-HCl (TLCK), L-1-chloro-3-[4-tosylamido]-4-phenyl-2-butanone (TPCK), tranexamic acid, glycyrrhizic acid, 18-β-glycyrrhetinic acid, colloidal oat extracts, elhibin, zinc salts, iodoacetate, phenylmethylsulfonyl fluoride, phosphoramidon, 4-(2-aminoethyl)-benzenesulfonylfluoride HCl, 3,4-dichloroiso-coumarin, quercetin, and the like, and mixtures thereof.

Chelating agents have also been found to be useful as inhibitors of both proteases and ureases at a concentration of about 0.1% to about 2%. Exemplary chelating agents are phytic acid, nitrilotriacetic acid, EDTA, diethylene triamino pentacetic acid, hyroxyethyl ethylene diamine triacetic acid, and the corresponding acid salts, disclosed in U.S. Patent No. 5,091,193 issued to Enjolras on February 25, 1992.

Among preferred protease inhibitors are compounds that exhibit inhibitory activity that is not necessarily restricted to a single class of proteases. Such compounds include, but are not limited to, hexamidine and its salts; pentamidine and its salts; benzamidine, p-aminobenzamidine and their derivatives; and guanidinobenzoic acid and its derivatives such as those disclosed in U.S. Patent No. 5,376,655 issued to Imaki et al. on December 27, 1994, the disclosure of which is hereby incorporated by reference. Other preferred protease inhibitors include polymer derivatives of guanidinobenzoic acid disclosed and made in U.S. Patent Application Serial No. 09/041196 entitled "Enzyme Inhibitors" filed in the name of McIver, et al. on March 12, 1998.

Protease inhibitors that are preferred in the practice are soybean trypsin inhibitor, aprotinin, hexamidine (e.g., hexamidine diisethionate), p-aminobenzamidine, leupeptin, pepstatin A, chymostatin and polymer derivatives of guanidinobenzoic acid (disclosed and made in U.S. Patent Application Serial No. 09/041196 entitled "Enzyme Inhibitors" incorporated by reference above). Particularly preferred protease inhibitors are soybean trypsin inhibitor, hexamidine, p-aminobenzamidine and the foregoing polymer derivatives of guanidinobenzoic acid.

Ureases are known to be inhibited in the presence of trace amounts of heavy metal ions, such as those of silver, copper, and the like. Thus, trace amounts (as little as 0.001% or less) of salts of these metals are useful as urease inhibitors. Other exemplary inhibitors of urease activity include, but are not limited to, acetyl hydroxamic acid and its derivatives, such as cinnamoyl hydroxamic acid and other alkyl hydroxamic acids; phosphoramidate and its derivatives. Such compounds are competitive inhibitors of urease at a concentration of about 2 micromolar (μM). Chelating agents have also been found to be useful as inhibitors of both proteases and ureases at a concentration of about 0.1% to about 2%. Exemplary chelating agents are phytic acid, nitrilotriacetic acid, ethylenediamine tetraacetic acid (EDTA), diethylene triamino pentacetic acid, hyroxyethyl ethylene diamine triacetic acid, and the corresponding acid salts, disclosed in U.S. Patent No. 5,091,193 incorporated by reference above. Other urease inhibiting compounds are disclosed in U.S. Patent 3,935,862 issued to Kraskin on February 3, 1976, and include amino acid compounds, such as hydroxyalkylamino acids, sulfhydryl amino acids, aminosulfonic acids, aminophosphonic acid compounds and ether amino acids such as methoxyethyliminodiacetic acid, ethylene-bis-(oxypropylaminodiacetic acid), ethylene-bis-(oxyethyliminodiacetic acid), amino-methyl phosphonic acid (N,N-diacetic acid), and the like, and aminopolycarboxylic acid compounds. including acids and salts diethylenetri-aminepentaacetic acid (DTPA), N-hydroxy-ethylethylenediaminetriacetic acid (HEDTA), and the like.

Other suitable inhibitors of urease are disclosed in U.S. Patent No. 5,409,903 issued to Polak et al. on April 25, 1995. This patent discloses dibasic magnesium phosphate, dialdehyde polysaccharides and zeolite, used alone in combination with each other or with the calcium compounds, calcium acetate, calcium chloride, calcium gluconate and calcium lactate as well as the magnesium compounds, magnesium chloride and magnesium citrate, for inhibition of ureases.

Suitable lipase inhibitors include, but are not limited to, water soluble salts of metals, such as cadmium, cobalt, copper, iron, molybdenum, silver, lanthanum, tin and zinc. Exemplary lipase inhibiting compounds are disclosed in U.S. Patent 4,556,560, and include zinc chloride, zinc acetate, zinc nitrate trihydrate, zinc nitrate hexahydrate, zinc sulfate, zinc sulfate heptahydrate, zinc sulfate hexahydrate, iron(II) chloride, iron(II) chloride tetrahydrate, iron(III) chloride, iron (III) chloride monohydrate, iron(III) chloride hexahydrate, iron(II) lactate, iron(III) lactate, iron(III) malate, iron(II) nitrate, iron(III) nitrate hexahydrate, iron(III) nitrate $9H_2O$, iron(II) sulfate and its hydrates, iron(III) sulfate and its hydrates, copper sulfate pentahydrate, tin chloride, cobalt chloride and lanthanum chloride, zinc salts of both saturated and unsaturated monocarboxylic acids having about 6 to about 12 carbon atoms, block copolymers of propylene oxide and ethylene oxide (e.g., marketed as Pluronic® and Tetronic® by BASF Corp.), glycerol triesters of fatty acids having from about 2 to about 20 carbons such as triacetin, and the like. Other useful lipase inhibitors are disclosed in U.S. Patent 5,091,193, hereby incorporated by reference, and include esters of fatty alcohols, such as saturated or unsaturated, linear or branched alkyl acetate, lactate or propionate containing 10 to 20 carbon atoms; saturated or unsaturated, linear or branched zinc salts of fatty acids of 2 to 22 carbon atoms, such as those formed with propionic acid isobutyric acid, caproic acid, undecylenic acid, and the like; zinc salts of aminated acylated acids, such as propionylcysteine, propionylhydroxyproline or caproylcysteine, and the like. Lipase inhibitors, such as the foregoing, have been found to be useful at a concentration of about 0.01 % to about 10%.

Still other useful lipase inhibitors, disclosed in European Patent Application Serial No. 97120699.0 entitled "Skin Rash Prevention Composition" filed in the name of Palumbo, et al. on Nobember 26, 1997 include specific ester compounds that act as a substitute substrate for fecal lipases and thereby are competitive lipase inhibitors. These esters have the formulas:

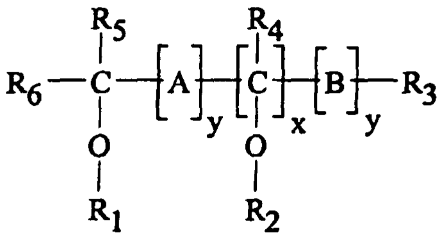

(I)

or

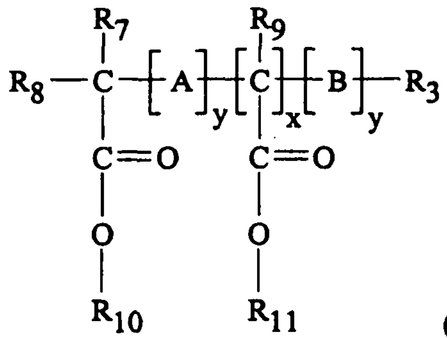

(II)

wherein $R_1$ and each $R_2$ independently are an acyl group with from 2 to 22 carbon atoms, or an alkyl, alkenyl, arylalkyl, hydroxyalkyl group with from 1 to 24 carbon atoms or hydrogen, whereby at least one of $R_1$ and $R_2$ is such an acyl group, $R_3$ $R_4$, $R_5$, $R_6$, $R_7$, $R_8$, and $R_9$ are independently an alkyl, alkenyl, arylalkyl, hydroxyalkyl, alkoxy groups of from 1 to 24 carbon atoms, hydroxy group or hydrogen; $R_{10}$ and $R_{11}$ are independently an alkyl, alkenyl, arylalkyl, hydroxyalkyl, alkoxy groups of from 2 to 24 carbon atoms, hydroxy group or hydrogen; A and B are independently a $C_1$-$C_6$ linear or branched alkylene, alkenylene, alkoxylene, hydroxyalkylene groups; the values of x are independently from 0 to 15; the values of y are independently 0 or 1, with the proviso that when x =2 and y=0, at least one $R_2$ is an alkyl, alkenyl, arylalkyl, hydroxyalkyl group with from 1 to 24 carbon atoms or hydrogen.

Still further examples of lipase inhibitors are those disclosed in U.S. patent 5,643,874 which include: (2S,3S , 5S)-5-[(S)-2-formamido-4-methyl-valeryloxy]-2-hexyl-3-hydroxy-hexadecanoic 1,3 acid lactone, also known as tetrahydrolipstatin; (2S ,3S,5S,7Z, 10Z)-5-[(S)-2-formamido-4-methyl-valeryloxy]-2-hexyl-3-hydroxy-7,10-hexadecadienoic 1,3 acid lactone, also known as lipstatin;1-(trans-4-isobutylcyclohexyl)-2-(phenylsulfonyloxy)ethanone, also known as FL-386; 4-methylpiperidine-1-carboxylic acid 4-phenoxyphenyl ester, also known as WAY-121898; N-[3-chloro-4-(trifluoromethyl)phenyl-]N'-[3-(trifluoromethyl)-phenyl]urea, also known as BAY-N-3176; N-formyl-L-valine-(S)-1 -[[(2S, 3S)-3-hexyl-4-oxo-2-oxetanyl]methyl]hexyl ester, also known as valilactone; (2S,3S,5S,7Z,10Z)-5-[(S)-2-acetamido-3-carbamoylpropionyloxy]-2-hexyl-3-hydroxy-7,10-hexadecadienoic lactone, also known as esterastin; (3S,4S)-4-[(1S,5R, 7S,8R,9RE)-8-hydroxy 1,3,5,7,9-pentamethyl-6-oxo-3-undecenyl]-3-methyl-2-oxetanone, also known as ebelactone A; (3S,4S)-3-ethyl-4-[(1S,5R,7S,8R,9R,E)-8-hydroxy-1,3,5,7,9-pentamethyl-6-oxo-3-undecenyl]-2-oxetanone, also known as ebelactone B; and 1,6-di(O-(carbamoyl)cyclohexanone oxime)hexane, also known as RHC 80267.

Exemplary inhibitors of bile salts that are coenzymes for lipolytic enzymes and are useful as lipase enzyme inhibitors in the absorbent articles include, but are not limited to, cationic compounds disclosed in European Patent Application Serial No. 97120700.6 entitled "Skin Care Composition" filed in the name of Palumbo, et al. on Nobember 26, 1997. Such compounds have the formulas:

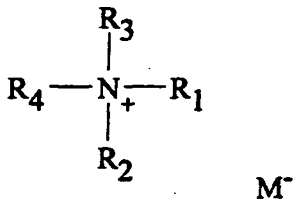

(I)

or

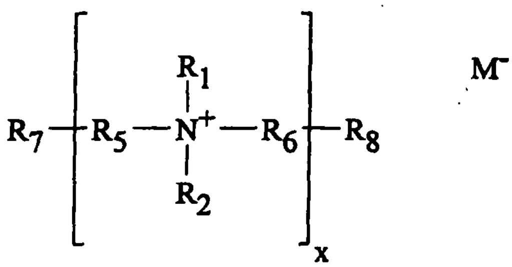

(II)

or an amphoteric compound and preferably an acidity source, the amphoteric compound having at its iso-electric point the formula:

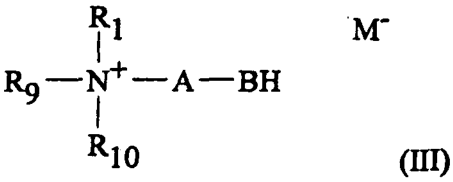

(III)

for preparation of a composition for treatment, prevention or reduction of lipolytic dermatitis of the external skin, wherein $R_1$, $R_2$, $R_3$ and $R_4$ are independently a $C_1$-$C_{22}$ alkyl, alkenyl, aryl, arylalkyl, amidoalkyl, (poly) alkoxy, hydroxyalkyl, or acyl groups, or two or more groups of $R_1$, $R_2$, $R_3$ and $R_4$ form together one or more ring structures; $R_5$, $R_6$ and A are independently a $C_1$-$C_{22}$ alkylene, alkenylene, (poly) alkoxylene, hydroxyalkylene, arylalkylene or amido alkylene groups; $R_7$ and $R_8$ are independently a $C_1$-$C_4$ alkyl, alkenyl, alkoxy group or a hydroxy group or hydrogen; $R_9$ and $R_{10}$ are independently a $C_1$-$C_{22}$ alkyl, alkenyl, aryl, arylalkyl, amidoalkyl, (poly) alkoxy, hydroxyalkyl, or acyl groups, or two or more of the groups $R_1$, $R_9$ and $R_{10}$ form together one or more ring structures; BH is a proton donating group; x is from 2 to 4; and $M^-$ is a counter ion.

Another exemplary suitable bile salt inhibitor is cholestyramine, described in a publication by C. Michael White et al., entitled "Cholestyramine Ointment to Treat Buttocks Rash and Anal Excoriation in an Infant", The Annals of Pharmacotherapy 30: 954-956, Sept. 1996.

Derivatives of p-guanidinobenzoic acid, especially esters of p-guanidinobenzoic acid, have been described as inhibitors of esterases. Such inhibitors are useful in the skin care compositions of the absorbent articles, and are disclosed in U.S. Patent 5,376,655 issued to Imaki et al. on December 27, 1994.

Suitable amylase inhibitors and/or glucosidase amylase inhibitors include those disclosed in U.S. patent 5,643,874, hereby incorporated by reference, and include O-4,6-dideoxy-4-[[[1S-(1α,4α,5β,6α)]-4,5,6-trihydroxy-3-(hydroxymethyl)-2-cyclohexen-1-yl]amino]-α-D-glucopyranosyl-(1→4)O-α-D-glucopyranosyl-(1→4)-D-glucose, also known as acarbose; 2(S),3(R),4(S),5(S)-tetrahydroxy-N-[2-hydroxy-1-(hydroxymethyl)-ethyl]-5-(hydroxymethyl)-1(S)-cyclohexamine, also known as voglibose;1,5-dideoxy-1,5-[(2-hydroxyethyl)imino]-D-glucitol, also known as miglitol; 1,5-dideoxy-1,5-[2-(4-ethoxycarbonylphenoxy)-ethylimino]-D-glucitol, also known as emiglitate; 2,6-dideoxy-2,6-imino-7-(β-D-glucopyranosyl)-D-glycero-L-guloheptitol, also known as MDL-25637; 1,5-dideoxy-1,5-(6-deoxy-1-O-methyl-α-D-glucopyranos-6-ylimino)-D-glucitol, also known as camiglibose;1,5,9,11,14-pentahydroxy-3-methyl-8,13-dioxo-5,6,8,13-tetrahydrobenzo[a]-naphthacene-2-carboxylic acid, also known pradimicin Q; also known as adiposine; and 1,2-dideoxy-2-[2(S),3(S),4(R)-trihydroxy-5-(hydroxymethyl)-5-cyclohexen-1(S)-ylamino]-L-glucopyranose, also known as salbostatin. Other suitable amylase inhibitors include tendamistat, trestatins, and those derived from plants, especially

from wheat, rice, maize, barley and other cereal grains, beans, and seaweed.

These and other features of enzyme inhibitors are disclosed in U.S. Patent Application Serial No. 09/041232 entitled "Protease Inhibitors in Absorbent Articles" filed in the name of Rourke, et al. on March 12, 1998, and U.S. Patent Application Serial No. 09/041266 entitled "Disposable Absorbent Article Having A Skin Care Composition Containing An Enzyme Inhibitor" filed in the name of Roe, et al. on March 12, 1998.

The skin care active ingredients are soluble or insoluble. Insoluble skin care active ingredients may be dispersed in skin care composition with dispersing agents to form homogeneous mixture of skin care composition. It is preferable that the insoluble skin care active ingredients are pre-dispersed in pre-dispersion solvents with dispersing agents. Pre-dispersion solvents may be petroleum-based, fatty acid ester, alkyl ethoxylate, fatty acid ester ethoxylates, fatty alcohol, polysiloxane, or mixtures of them. Further, pre-dispersion solvents may be soluble skin care active ingredients. Exemplary preferable dispersing agents are diethanolamine polyoxyethylene oleyl ether phosphate, polyhydroxystearic acid, polyglyceryl - 6 polyricinoleate, neopentyl glycol diisostearate, propylene glycol dicaprate, or mixtures thereof. Other possible dispersing agents are isoelcosane and polyisobutene and quaternium 18, phenyltrimethicone and quaternium - 18 hectorite and triethyl citrate, isohexadecane and quaternium - 18 hectorite and propylene carbonate, octyldodecanol and quaternium - 18 hectorite and propylene carbonate, mineral oil and quaternium - 18 hectorite and propylene carbonate, isopropyl myristate and stearalkonium hectorite and propylene carbonate, cyclomethicone and quaternium - 18 and SDA 40, lanolin oil and isopropyl palmitate and stearalkonium hectorite and propylene carbonate and propyl paraben, 1 - eicosanol, or mixtures thereof.

4. <u>Other Optional Component</u>

Skin care compositions may comprise other optional components. These optional components include water, hydrophilic surfactants, viscosity modifiers, perfumes, film formers, deodorants, opacifiers, solvents, and the like. Stabilizers may be added to enhance the shelf life of the skin care composition such as cellulose derivatives, proteins and lecithin. Hydrophilic surfactants may be added to promote rapid transfer of the fluid (e.g., urine, menses etc.) from the topsheet to the absorbent inner layers of the article. The detail of such hydrophilic surfactant is disclosed in, e.g., U.S. Patent 5,643,588, "Diaper Having a Lotioned Topsheet", issued to Roe, Bakes & Warner on July 1, 1997.

<u>C. Treating Body Contacting Surface With Skin Care Composition</u>

In preparing products treated with skin care compositions, the skin care composition is applied onto at least a portion of the body facing surface of absorbent articles. Any of a variety of application methods that evenly distribute viscous materials can be used. Suitable methods include printing, spraying, coating, brushing, extrusion, or combinations of these application techniques.

The minimum level of skin care composition to be applied onto the body contacting surface of the article is an amount effective for providing the skin effects to the wearer. The level of skin care compositions applied will depend on various factors, including the relative amount of surface area of the body contacting surface not treated with skin care composition, expected skin effects of the skin care composition and the like. The amount of the skin care composition can also vary over the body contacting surface. For example, some portions of the body contacting surface can have greater or lesser amounts of skin care composition than the other portions of the body contacting surface, including portions of the surface that do not have any skin care composition on it.

The skin care composition can be applied onto the entire surface of the body contacting surface or selectively onto the portions of the body contacting surface. For example, if the skin care composition is applied to the topsheet of absorbent article, the way of applying the skin care composition onto the topsheet of absorbent articles is preferable such that the skin care composition is distributed primarily on body facing part of the topsheet. However, if the skin care composition is relatively hydrophobic, it is preferable that the skin care composition is not distributed in the fluid path (such as apertures of the topsheet) and does not block the fluid path to ensure the ability of the topsheet to transmit fluid to the underlying absorbent core. Examples of the pattern of the coating include stripes, dots, circles or the like.

The skin care composition can be applied to the body contacting surface at any point during assembly. For example, the skin care composition can be applied to the body contacting surface of the finished absorbent article before it has been packaged. The skin care composition can also be applied to the body contacting surface before it is combined with the other raw materials to form the finished absorbent article.

The skin care composition is typically applied from a melt thereof to the article. Since the skin care composition melts at significantly above ambient temperatures, it is usually applied as a heated coating to the body contacting surface of the article. The temperature is determined considering primarily the melting point of the skin care composition and the other factors such as lowering of the temperature in the manufacturing process of the article. And the skin care composition is often heated to a temperature in the range from 50°C to 100°C, more often from 60°C to 90°C, prior to being applied to the article. Once the melted skin care composition has been applied to the article, it is allowed to cool

and solidify to form solidified coating or film on the surface of the body contacting surface. Preferably, the application process is designed to aid in the cooling/set up of the skin care composition. Examples of applying the skin care composition to the body contacting surface is described in, e.g., U.S. Patent 5,643,588, "Diaper Having a Lotioned Topsheet", issued to Roe, Bakes & Warner on July 1, 1997.

SPECIFIC ILLUSTRATIONS OF THE PREPARATION OF TREATED ARTICLE ACCORDING TO THE PRESENT INVENTION

The following examples further describe and demonstrate the preferred embodiments within the scope of the present invention.

Example 1

A. Preparation of Skin Care Compositions

A water free skin care composition (Skin Care Composition A) is made by mixing the following melted components together: Petrolatum made by Witco Corp. under the name Super White Protopet®, and Behenyl Alcohol made by Henkel Corp. under the name Lanette 22. Super White Protopet® is petrolatum. The ratio of the heavy aliphatic hydrocarbons having alkyl chain length of from 27 to 36 to the light hydrocarbons having alkyl chain length of from 17 to 26 is 2.3:1.0. The ratio of the heavy aliphatic hydrocarbons having alkyl chain length of from 30 to 36 to the light aliphatic hydrocarbons aliphatic having alkyl chain length of from 20 to 26 is 1.7:1.0. The ratio of the heavy aliphatic hydrocarbons having alkyl chain length of from 33 to 36 to the light aliphatic hydrocarbons aliphatic having alkyl chain length of from 23 to 26 is 1.3:1.0. Lanette 22 is a mixed linear $C_{18}$-$C_{24}$ primary alcohol. Lanette 22 contains about 70% of $C_{22}$ fatty alcohol, about 20 % of $C_{20}$ alcohol, about 9% of $C_{18}$ alcohol, and about 1 % of $C_{24}$ fatty alcohol. The weight percentages of these components are shown in Table I below:

Table I

| Component | Weight % |
|---|---|
| Super White Protopet® | 70 |
| Behenyl Alcohol | 30 |

B. Preparation of Skin Care Composition Treated Article bv Gravure Printing

Skin Care Composition A is placed into a heated tank operating at a temperature of 90°C. Skin Care Composition A is subsequently applied with a gravure printer made by Inoue Kinzoku Kogyo Co. Ltd, Shiga, Japan , operating at a roll temperature of 90°C and a roll pressure of 11.5g/cm$^2$ on a preferential acquisition zone of the DRI-WEAVE topsheet and the reminder (i.e., skin care zone) of the DRI-WEAVE topsheet of Whisper Slim Wing Regular manufactured by Procter & Gamble Far East, Inc. (Absorbent Article A). I.e., Skin Care Composition A is applied on the DRI-WEAVE topsheet of the oval-shaped preferential acquisition zone having a major axis (longitudinal length) of 80 mm and a minor axis (transverse width) of 40mm at an add-on level = 2 g/m$^2$. The preferential acquisition zone positions at the center of Absorbent Article A such that the major axis aligns with the longitudinal center line of Absorbent Article A and the minor axis aligns with the transverse center line of Absorbent Article A. Skin Care Composition A is also applied on the reminder of the DRI-WEAVE topsheet of Absorbent Article A at an add-on level = 30 g/m$^2$.

Example 2

A. Preparation of Skin Care Compositions

Skin Care Composition A is prepared in accordance with the description above.

B. Preparation of Skin Care Composition Treated Article bv Gravure Printing

Skin Care Composition A is placed into a heated tank operating at a temperature of 90°C. Skin Care Composition A is subsequently applied with a gravure printer made by Inoue Kinzoku Kogyo Co. Ltd, Shiga, Japan, operating at a roll temperature of 90°C and a roll pressure of 11.5g/cm$^2$ on a preferential acquisition zone of the DRI-WEAVE topsheet and the reminder (i.e., skin care zone) of the DRI-WEAVE topsheet of Whisper Slim Wing Regular manufactured by

Procter & Gamble Far East, Inc. (Absorbent Article B). I.e., Skin Care Composition A is applied on the DRI-WEAVE topsheet of the oval-shaped preferential acquisition zone having a major axis (longitudinal length) of 100 mm and a minor axis (transverse width) of 50 mm at an add-on level = 4 g/$m^2$. The preferential acquisition zone positions at the center of Absorbent Article B such that the major axis aligns with the longitudinal center line of Absorbent Article B and the minor axis aligns with the transverse center line of Absorbent Article A. Skin Care Composition A is also applied on the reminder of the DRI-WEAVE topsheet of Absorbent Article B at an add-on level = 40 g/$m^2$.

Example 3

A. Preparation of Skin Care Compositions

Skin Care Composition A is prepared in accordance with the description above:

B. Preparation of Skin Care Composition Treated Article by Hot Melt Printing

Skin Care Composition A is placed into a heated tank operating at a temperature of 90°C. Skin Care Composition A is subsequently applied with a contact applicator using a Meltex EP45 hot melt adhesive applicator head operating at a temperature of 90°C on a preferential acquisition zone of the DRI-WEAVE topsheet and the reminder (i.e., skin care zone) of the DRI-WEAVE topsheet of Whisper Slim Wing Regular manufactured by Procter & Gamble Far East, Inc. (Absorbent Article C). I.e., Skin Care Composition A is applied on the DRI-WEAVE topsheet of the rectangle-shaped preferential acquisition zone having longitudinal length of 120 mm and latitudinal width of 50 mm at an add-on level = 2 g/$m^2$. The preferential acquisition zone positions at the center of Absorbent Article C such that aligns with the longitudinal center line of Absorbent Article C and the minor axis aligns with the transverse center line of Absorbent Article C. Skin Care Composition A is also applied on the reminder of the DRI-WEAVE topsheet of Absorbent Article C at an add-on level = 40 g/$m^2$.

Example 4 (not part of the invention)

A. Preparation of Skin Care Compositions

Skin Care Composition A is prepared in accordance with the description above.

B. Preparation of Skin Care Composition Treated Article by Gravure Printing

Skin Care Composition A is placed into a heated tank operating at a temperature of 90°C. Skin Care Composition A is subsequently applied with a gravure printer made by Inoue Kinzoku Kogyo Co. Ltd, Shiga, Japan, operating at a roll temperature of 90°C and a roll pressure of 11.5g/$cm^2$ on the entirety of the DRI-WEAVE topsheet (i.e., skin care zone) other than the preferential acquisition zone of the DRI-WEAVE topsheet of Whisper Slim Wing Regular manufactured by Procter & Gamble Far East, Inc. (Absorbent Article D). I.e., Skin Care Composition A is not applied on the DRI-WEAVE topsheet of the oval-shaped preferential acquisition zone having a major axis (longitudinal length) of 80 mm and a minor axis (transverse width) of 40 mm. The preferential acquisition zone positions at the center of Absorbent Article D such that the major axis aligns with the longitudinal center line of Absorbent Article D and the minor axis aligns with the transverse center line of Absorbent Article D. Skin Care Composition A is also applied on the reminder of the DRI-WEAVE topsheet of Absorbent Article D at an add-on level = 30 g/$m^2$.

Example 5

A. Preparation of Skin Care Compositions

A water free skin care composition (Skin Care Composition B) is made by mixing the following melted components together: Petrolatum made by Witco Corp. under the name Protopet® 1S, and Behenyl Alcohol made by Henkel Corp. under the name Lanette 22. Protopet® 1S is petrolatum. The ratio of the heavy aliphatic hydrocarbons having alkyl chain length of from 27 to 36 to the light hydrocarbons having alkyl chain length of from 17 to 26 is 2.3:1.0. The ratio of the heavy aliphatic hydrocarbons having alkyl chain length of from 30 to 36 to the light aliphatic hydrocarbons aliphatic having alkyl chain length of from 20 to 26 is 1.7:1.0. The ratio of the heavy aliphatic hydrocarbons having alkyl chain length of from 33 to 36 to the light aliphatic hydrocarbons aliphatic having alkyl chain length of from 23 to 26 is 1.3:1.0. Lanette 22 contains about 70% of $C_{22}$ fatty alcohol, about 20 % of $C_{20}$ alcohol, about 9 % of $C_{18}$ alcohol, and about 1 % of $C_{24}$ fatty alcohol. The weight percentages of these components are shown in Table II below:

Table II

| Component | Weight % |
|---|---|
| Protopet® 1S | 80 |
| Behenyl Alcohol | 20 |

B. Preparation of Skin Care Composition Treated Article by Gravure Printing

Skin Care Composition B is placed into a heated tank operating at a temperature of 90°C. Skin Care Composition B is subsequently applied with a gravure printer made by Inoue Kinzoku Kogyo Co. Ltd, Shiga, Japan, operating at a roll temperature of 90°C and a roll pressure of 11.5g/cm$^2$ on a preferential acquisition zone of the DRI-WEAVE topsheet and the flap skin care zone, the periphery skin care zone, the front skin care zone and the back skin care zone of the DRI-WEAVE topsheet of Whisper Slim Wing Regular manufactured by Procter & Gamble Far East, Inc. (Absorbent Article E). I.e., Skin Care Composition B is applied on the DRI-WEAVE topsheet of the oval-shaped preferential acquisition zone having a major axis (longitudinal length) of 80 mm and a minor axis (transverse width) of 40 mm at an add-on level = 2 g/m$^2$. The preferential acquisition zone positions at the center of Absorbent Article E such that the major axis aligns with the longitudinal center line of Absorbent Article E and the minor axis aligns with the transverse center line of Absorbent Article E. Skin Care Composition B is applied on the flap skin care zone, the periphery skin care zone, the front skin care zone, and the back skin care zone of the DRI-WEAVE topsheet of Absorbent Article E at an add-on level = 40 g/m$^2$, 40 g/m$^2$, 20 g/m$^2$, and 20 g/m$^2$, respectively.

Example 6

A. Preparation of Skin Care Compositions

Skin Care Composition B is prepared in accordance with the description above.

B. Preparation of Skin Care Composition Treated Article by Gravure Printing

Skin Care Composition B is placed into a heated tank operating at a temperature of 90°C. Skin Care Composition B is subsequently applied with a gravure printer made by Inoue Kinzoku Kogyo Co. Ltd, Shiga, Japan, operating at a roll temperature of 90°C and a roll pressure of 11.5g/cm$^2$ on a preferential acquisition zone of the DRI-WEAVE topsheet and the reminder of the DRI-WEAVE topsheet (including the flap skin care zone, the periphery skin care zone, the front skin care zone and the back skin care zone) of Whisper Slim Wing Regular manufactured by Procter & Gamble Far East, Inc. (Absorbent Article F). I.e., Skin Care Composition B is applied on the DRI-WEAVE topsheet of the oval-shaped preferential acquisition zone having a major axis (longitudinal length) of 80 mm and a minor axis (transverse width) of 40 mm at an add-on level = 4 g/m$^2$. The preferential acquisition zone positions at the center of Absorbent Article F such that the major axis aligns with the longitudinal center line of Absorbent Article F and the minor axis aligns with the transverse center line of Absorbent Article F. Skin Care Composition B is applied on the flap skin care zone, the periphery skin care zone, the front skin care zone, and the back skin care zone of the DRI-WEAVE topsheet of Absorbent Article F at an add-on level = 60 g/m$^2$, 60 g/m$^2$, 30 g/m$^2$, and 30 g/m$^2$, respectively.

Example 7

A. Preparation of Skin Care Compositions

A water free skin care composition (Skin Care Composition C) is made by mixing zinc oxide made by Zinc Corporation of America under the name USP-1 and fumed silica made by Cabot Corp. under the name CAB-O-Sil® TS-720 and the following melted components; Petrolatum made by Witco Corp. under the name Super White Protopet®, and Behenyl Alcohol made by Henkel Corp. under the name Lanette 22. These are mixed by a high speed blade mixer (Tokusyu Kika TK Robo Mics, operating at 5000rpm) at 90°C. Super White Protopet® is petrolatum. The ratio of the heavy aliphatic hydrocarbons having alkyl chain length of from 27 to 36 to the light hydrocarbons having alkyl chain length of from 17 to 26 is 2.3:1.0. The ratio of the heavy aliphatic hydrocarbons having alkyl chain length of from 30 to 36 to the light aliphatic hydrocarbons aliphatic having alkyl chain length of from 20 to 26 is 1.7:1.0. The ratio of the heavy aliphatic hydrocarbons having alkyl chain length of from 33 to 36 to the light aliphatic hydrocarbons aliphatic having alkyl chain length of from 23 to 26 is 1.3:1.0. Lanette 22 contains about 70% of $C_{22}$ fatty alcohol, about 20 % of $C_{20}$ alcohol,

about 9 % of $C_{18}$ alcohol, and about 1 % of $C_{24}$ fatty alcohol. USP-1 is zinc oxide having an average particle diameter of about 0.1-1.0 μm. CAB-O-Sil® TS-720 is fumed silica whose surface is treated with polydimethylsiloxane. The weight percentages of the components are shown in Table III below:

Table III

| Component | Weight % |
|---|---|
| Super White Protopet® | 67 |
| Behenyl Alcohol | 30 |
| Fumed Silica | 1.0 |
| Zinc Oxide | 2.0 |

B. Preparation of Skin Care Composition Treated Article by Gravure Printing

Skin Care Composition C is placed into a heated tank operating at a temperature of 90°C. Skin Care Composition C is subsequently applied with a gravure printer made by Inoue Kinzoku Kogyo Co. Ltd, Shiga, Japan, operating at a roll temperature of 90°C and a roll pressure of 11.5g/$cm^2$ on a preferential acquisition zone of the DRI-WEAVE topsheet of Whisper Slim Wing Regular manufactured by Procter & Gamble Far East, Inc. (Absorbent Article G) and the reminder (i.e., skin care zone) of the DRI-WEAVE topsheet of Absorbent Article G. I.e., Skin Care Composition C is applied on the DRI-WEAVE topsheet of the oval-shaped preferential acquisition zone having a major axis (longitudinal length) of 80 mm and a minor axis (transverse width) of 40 mm at an add-on level = 2 g/$m^2$. The preferential acquisition zone positions at the center of Absorbent Article G such that the major axis aligns with the longitudinal center line of Absorbent Article G and the minor axis aligns with the transverse center line of Absorbent Article G. Skin Care Composition C is also applied on the reminder of the DRI-WEAVE topsheet of Absorbent Article G at an add-on level = 30 g/$m^2$.

Example 8 (not part of the invention)

A. Preparation of Skin Care Compositions

Skin Care Composition C is prepared in accordance with the description above.

B. Preparation of Skin Care Composition Treated Article by Gravure Printing

Skin Care Composition C is placed into a heated tank operating at a temperature of 90°C. Skin Care Composition C is subsequently applied with a gravure printer made by Inoue Kinzoku Kogyo Co. Ltd, Shiga, Japan, operating at a roll temperature of 90°C and a roll pressure of 11.5g/$cm^2$ on the entirety of the DRI-WEAVE topsheet (i.e., skin care zone) other than the preferential acquisition zone of the DRI-WEAVE topsheet of Whisper Slim Wing Regular manufactured by Procter & Gamble Far East, Inc. (Absorbent Article H). I.e., Skin Care Composition C is not applied on the DRI-WEAVE topsheet of the oval-shaped preferential acquisition zone having a major axis (longitudinal length) of 80 mm and a minor axis (transverse width) of 40 mm. The preferential acquisition zone positions at the center of Absorbent Article H such that the major axis aligns with the longitudinal center line of Absorbent Article H and the minor axis aligns with the transverse center line of Absorbent Article H. Skin Care Composition C is also applied on the reminder of the DRI-WEAVE topsheet of Absorbent Article H at an add-on level = 30 g/$m^2$.

It should also be understood that all of the limits and ranges specified herein include all narrower ranges, limits, and amounts that are within the specified limits and ranges and that such narrower ranges and limits may be claimed even though those limits and ranges are not separately listed.

While particular embodiments of the present invention have been illustrated and described, it would be obvious to those skilled in the art that various other changes and modifications can be made without departing from the spirit and scope of the invention.

## Claims

1. A feminine hygiene absorbent article having a body surface and a garment surface, the absorbent article comprising a topsheet disposed at the body surface, a backsheet disposed at the garment surface, and an absorbent core disposed therebetween, at least a portion of the absorbent article being provided with a skin care composition

comprising one or more hydrophobic emollient(s), **characterized in that** the absorbent article has a preferential acquisition zone and a skin care zone, the preferential acquisition zone covering at least a portion of the vulva of the wearer when the absorbent article is applied on the wearer's body, wherein both said preferential acquisition zone and said skin care zone are provided with skin care composition, the skin care zone is provided with the skin care composition of greater basis weight than the preferential acquisition zone.

2. The feminine hygiene absorbent article of Claim 1 wherein the preferential acquisition zone is provided with the skin care composition of not greater than 20 $g/m^2$, preferably not greater than 12 $g/m^2$, more preferably not greater than 4 $g/m^2$.

3. The feminine hygiene absorbent article of Claim 1 wherein the skin care composition is provided on the topsheet of the preferential acquisition zone, wherein the amount of skin care composition is selected such that the acquisition rate of the portion of the topsheet which is provided with the skin care composition is not less than 70% of the acquisition rate of the topsheet before the skin care composition is applied, preferable not less than 75% of the acquisition rate of the topsheet before the skin care composition is applied, more preferably not less than 85% of the acquisition rate of the topsheet before the skin care composition is applied.

4. The feminine hygiene absorbent article of Claim 1 wherein at least a portion of the skin care zone is provided with a skin care composition of between 0.1 $g/m^2$ and 100 $g/m^2$, preferably between 0.5 $g/m^2$ and 90 $g/m^2$, more preferably between 1 $g/m^2$ and 80 $g/m^2$.

5. The feminine hygiene absorbent article of Claim 1 wherein the absorbent article has a main body portion, the main body portion having at least three sections, the three sections comprising a first end section, a second end section, and a central section which is disposed between the first end section and the second end section, wherein the preferential acquisition zone includes at least a portion of the central section.

6. The feminine hygiene absorbent article of Claim 5 wherein the skin care zone includes at least a portion of the first end section or at least a portion of the second end section.

7. The feminine absorbent article of Claim 6 wherein the preferential acquisition zone is defined by a transverse width and a longitudinal length, wherein the width is between 10 mm and 80 mm and the length is between 20 mm and 120 mm, preferably the width is between 20 mm and 70 mm and the length is between 30 mm and 110 mm, more preferably the width is between 30 mm and 60 mm and the length is between 40 mm and 100 mm.

8. The feminine hygiene absorbent article of Claim 1 wherein the absorbent article has flaps extending laterally outwardly, wherein the skin care zone includes a portion of the flap which is folded during the use of the absorbent article.

9. The feminine hygiene absorbent article of Claim 1 wherein the absorbent article has a peripheral portion to define the outline of the absorbent article, wherein the skin care zone includes at least a portion of the peripheral portion.

## Patentansprüche

1. Absorptionsartikel für die weibliche Intimhygiene mit einer körperseitigen Oberfläche und einer kleidungsseitigen Oberfläche, wobei der Absorptionsartikel eine Oberschicht, die an der körperseitigen Oberfläche angeordnet ist, eine Unterschicht, die an der kleidungsseitigen Oberfläche angeordnet ist, und einen dazwischen angeordneten Absorptionskern umfasst, wobei mindestens ein Teil des Absorptionsartikels mit einer Hautpflegezusammensetzung versehen ist, die einen oder mehrere hydrophobe Weichmacher umfasst, **dadurch gekennzeichnet, dass** der Absorptionsartikel eine bevorzugte Aufnahmezone und eine Hautpflegezone aufweist, wobei die bevorzugte Aufnahmezone mindestens einen Teil der Vulva der Trägerin bedeckt, wenn der Absorptionsartikel an den Körper der Trägerin angelegt wird, wobei sowohl die bevorzugte Aufnahmezone als auch die Hautpflegezone mit einer Hautpflegezusammensetzung versehen sind, die Hautpflegezone zu einem größeren Flächengewicht mit der Hautpflegezusammensetzung versehen ist als die bevorzugte Aufnahmezone.

2. Absorptionsartikel für die weibliche Intimhygiene nach Anspruch 1, wobei die bevorzugte Aufnahmezone zu nicht mehr als 20 $g/m^2$, vorzugsweise zu nicht mehr als 12 $g/m^2$, mehr bevorzugt zu nicht mehr als 4 $g/m^2$ mit der

Hautpflegezusammensetzung versehen ist.

3. Absorptionsartikel für die weibliche Intimhygiene nach Anspruch 1, wobei die Hautpflegezusammensetzung auf der Oberschicht der bevorzugten Aufnahmezone bereitgestellt ist, wobei die Menge der Hautpflegezusammensetzung so ausgewählt ist, dass die Aufnahmegeschwindigkeit des Teils der Oberschicht, der mit der Hautpflegezusammensetzung versehen ist, nicht weniger als 70 % der Aufnahmegeschwindigkeit der Oberschicht vor dem Auftragen der Hautpflegezusammensetzung, vorzugsweise nicht weniger als 75 % der Aufnahmegeschwindigkeit der Oberschicht vor dem Auftragen der Hautpflegezusammensetzung, mehr bevorzugt nicht weniger als 85 % der Aufnahmegeschwindigkeit der Oberschicht vor dem Auftragen der Hautpflegezusammensetzung beträgt.

4. Absorptionsartikel für die weibliche Intimhygiene nach Anspruch 1, wobei mindestens ein Teil der Hautpflegezone mit einer Hautpflegezusammensetzung zwischen 0,1 $g/m^2$ und 100 $g/m^2$, vorzugsweise zwischen 0,5 $g/m^2$ und 90 $g/m^2$, mehr bevorzugt zwischen 1 $g/m^2$ und 80 $g/m^2$ versehen ist.

5. Absorptionsartikel für die weibliche Intimhygiene nach Anspruch 1, wobei der Absorptionsartikel ein Grundelement aufweist, wobei das Grundelement mindestens drei Abschnitte aufweist, wobei die drei Abschnitte einen ersten Endabschnitt, einen zweiten Endabschnitt und einen Mittelabschnitt, der zwischen dem ersten Endabschnitt und dem zweiten Endabschnitt angeordnet ist, umfassen, wobei die bevorzugte Aufnahmezone mindestens einen Teil des Mittelabschnitts umfasst.

6. Absorptionsartikel für die weibliche Intimhygiene nach Anspruch 5, wobei die Hautpflegezone mindestens einen Teil des ersten Endabschnitts oder mindestens einen Teil des zweiten Endabschnitts umfasst.

7. Absorptionsartikel für die weibliche Intimhygiene nach Anspruch 6, wobei die bevorzugte Aufnahmezone durch eine quer verlaufende Breite und eine längs verlaufende Länge definiert ist, wobei die Breite zwischen 10 mm und 80 mm beträgt und die Länge zwischen 20 mm und 120 mm beträgt, vorzugsweise die Breite zwischen 20 mm und 70 mm beträgt und die Länge zwischen 30 mm und 110 mm, mehr bevorzugt die Breite zwischen 30 mm und 60 mm und die Länge zwischen 40 mm und 100 mm beträgt.

8. Absorptionsartikel für die weibliche Intimhygiene nach Anspruch 1, wobei der Absorptionsartikel Flügel aufweist, die sich seitlich nach außen erstrecken, wobei die Hautpflegezone einen Teil des Flügels umfasst, der während des Gebrauchs des Absorptionsartikels umgelegt ist.

9. Absorptionsartikel für die weibliche Intimhygiene nach Anspruch 1, wobei der Absorptionsartikel einen peripheren Teil aufweist, der den Umfang des Absorptionsartikels definiert, wobei die Hautpflegezone mindestens einen Teil des peripheren Teils umfasst.

**Revendications**

1. Article absorbant d'hygiène féminine ayant une surface corporelle et une surface de vêtement, l'article absorbant comprenant une feuille de dessus disposée à la surface corporelle, une feuille de fond disposée à la surface de vêtement, et une âme absorbante disposée entre elles, au moins une partie de l'article absorbant étant pourvue d'une composition de soin de la peau comprenant un ou plusieurs émollient(s) hydrophobe(s), **caractérisé en ce que** l'article absorbant a une zone de recueil préférentiel et une zone de soin de la peau, la zone de recueil préférentiel couvrant au moins une partie de la vulve de l'utilisatrice lorsque l'article absorbant est appliqué sur le corps de l'utilisatrice, dans lequel tant ladite zone de recueil préférentiel que ladite zone de soin de la peau sont pourvues d'une composition de soin de la peau,
la zone de soin de la peau est pourvue de la composition de soin de la peau de plus grande masse surfacique que la zone de recueil préférentiel.

2. Article absorbant d'hygiène féminine selon la revendication 1, dans lequel la zone de recueil préférentiel est pourvue de la composition de soin de la peau à raison de pas plus de 20 $g/m^2$, de préférence pas plus de 12 $g/m^2$, plus préférablement pas plus de 4 $g/m^2$.

3. Article absorbant d'hygiène féminine selon la revendication 1, dans lequel la composition de soin de la peau est fournie sur la feuille de dessus de la zone de recueil préférentiel, dans lequel la quantité de composition de soin de la peau est choisie de telle sorte que la vitesse de recueil de la partie de la feuille de dessus qui est pourvue de la

composition de soin de la peau n'est pas inférieure à 70 % de la vitesse de recueil de la feuille de dessus avant application de la composition de soin de la peau, de préférence pas inférieure à 75 % de la vitesse de recueil de la feuille de dessus avant application de la composition de soin de la peau, plus préférablement pas inférieure à 85 % de la vitesse de recueil de la feuille de dessus avant application de la composition de soin de la peau.

**4.** Article absorbant d'hygiène féminine selon la revendication 1, dans lequel au moins une partie de la zone de soin de la peau est pourvue d'une composition de soin de la peau à raison d'entre 0,1 $g/m^2$ et 100 $g/m^2$, de préférence entre 0,5 $g/m^2$ et 90 $g/m^2$, plus préférablement entre 1 $g/m^2$ et 80 $g/m^2$.

**5.** Article absorbant d'hygiène féminine selon la revendication 1, où l'article absorbant a une partie du corps principale, la partie du corps principale ayant au moins trois sections, les trois sections comprenant une première section d'extrémité, une deuxième section d'extrémité et une section centrale qui est disposée entre la première section d'extrémité et la deuxième section d'extrémité, dans lequel la zone de recueil préférentiel inclut au moins une partie de la section centrale.

**6.** Article absorbant d'hygiène féminine selon la revendication 5, dans lequel la zone de soin de la peau inclut au moins une partie de la première section d'extrémité ou au moins une partie de la deuxième section d'extrémité.

**7.** Article absorbant féminin selon la revendication 6, dans lequel la zone de recueil préférentiel est définie par une largeur transversale et une longueur longitudinale, dans lequel la largeur est comprise entre 10 mm et 80 mm et la longueur est comprise entre 20 mm et 120 mm, de préférence la largeur est comprise entre 20 mm et 70 mm et la longueur est comprise entre 30 mm et 110 mm, plus préférablement la largeur est comprise entre 30 mm et 60 mm et la longueur est comprise entre 40 mm et 100 mm.

**8.** Article absorbant d'hygiène féminine selon la revendication 1, où l'article absorbant a des rabats s'étendant latéralement vers l'extérieur, dans lequel la zone de soin de la peau inclut une partie du rabat qui est pliée durant l'utilisation de l'article absorbant.

**9.** Article absorbant d'hygiène féminine selon la revendication 1, où l'article absorbant a une partie périphérique pour définir le contour de l'article absorbant, dans lequel la zone de soin de la peau inclut au moins une partie de la partie périphérique.

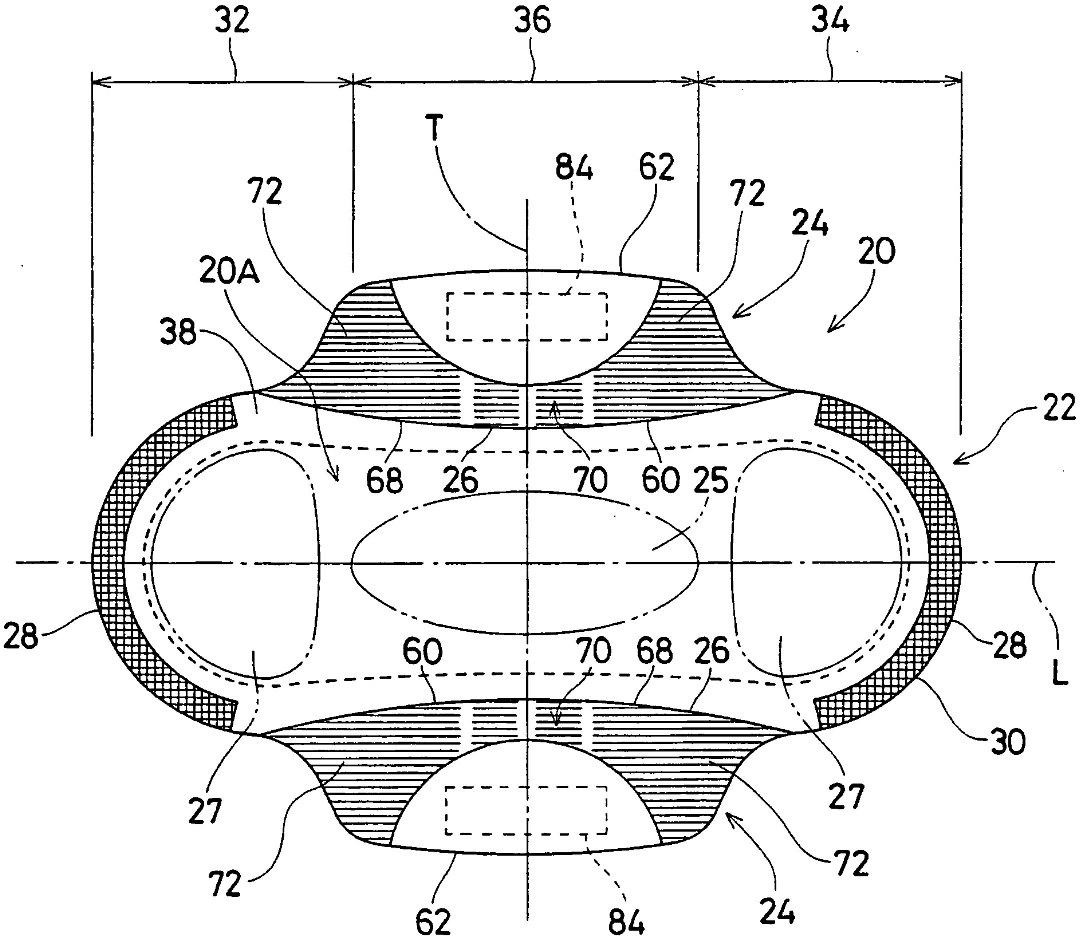
32
36
34
T
84
62
72
72
24
20
20A
38
22
68
26
70
60
25
28
28
L
60
70
68
26
30
27
27
72
72
62
84
24

FIG. 1

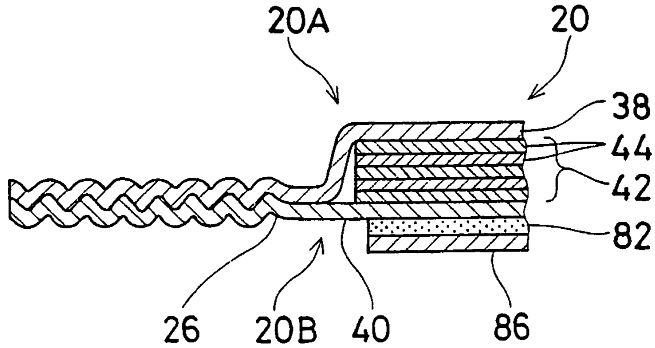
20A
20
38
44
42
82
26
20B
40
86

FIG. 2

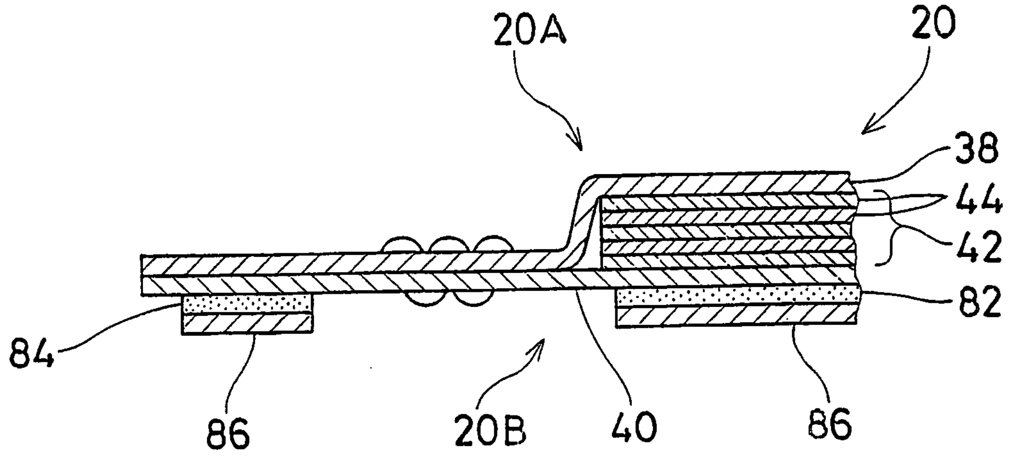


FIG. 3

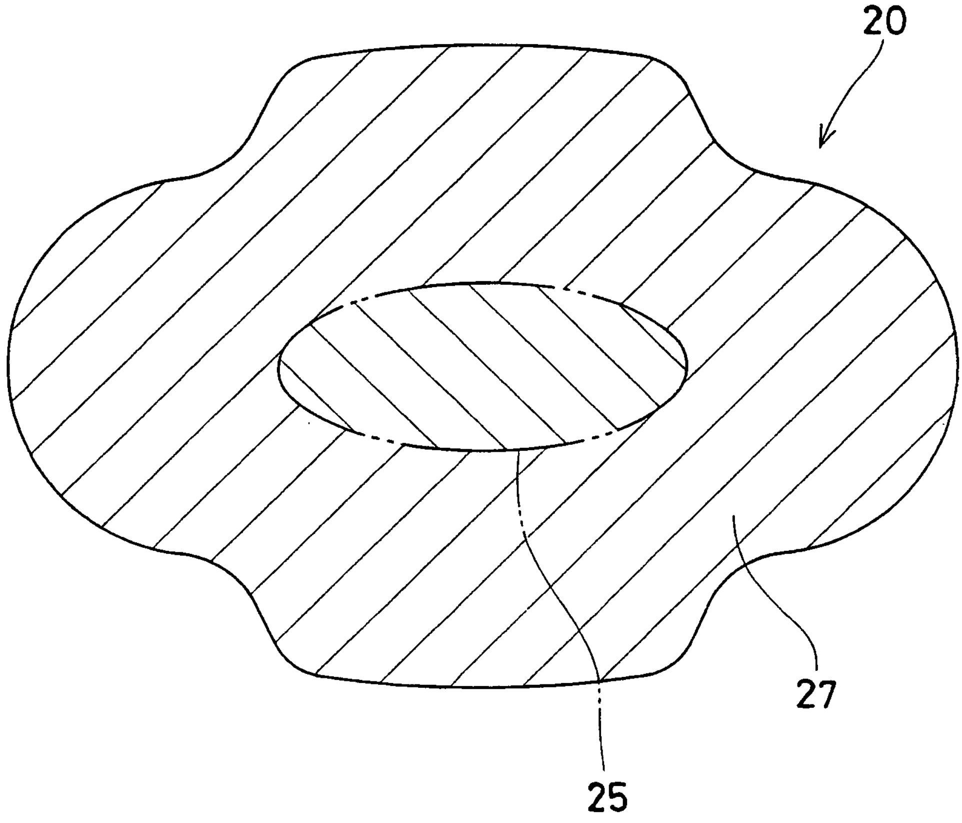


FIG. 4

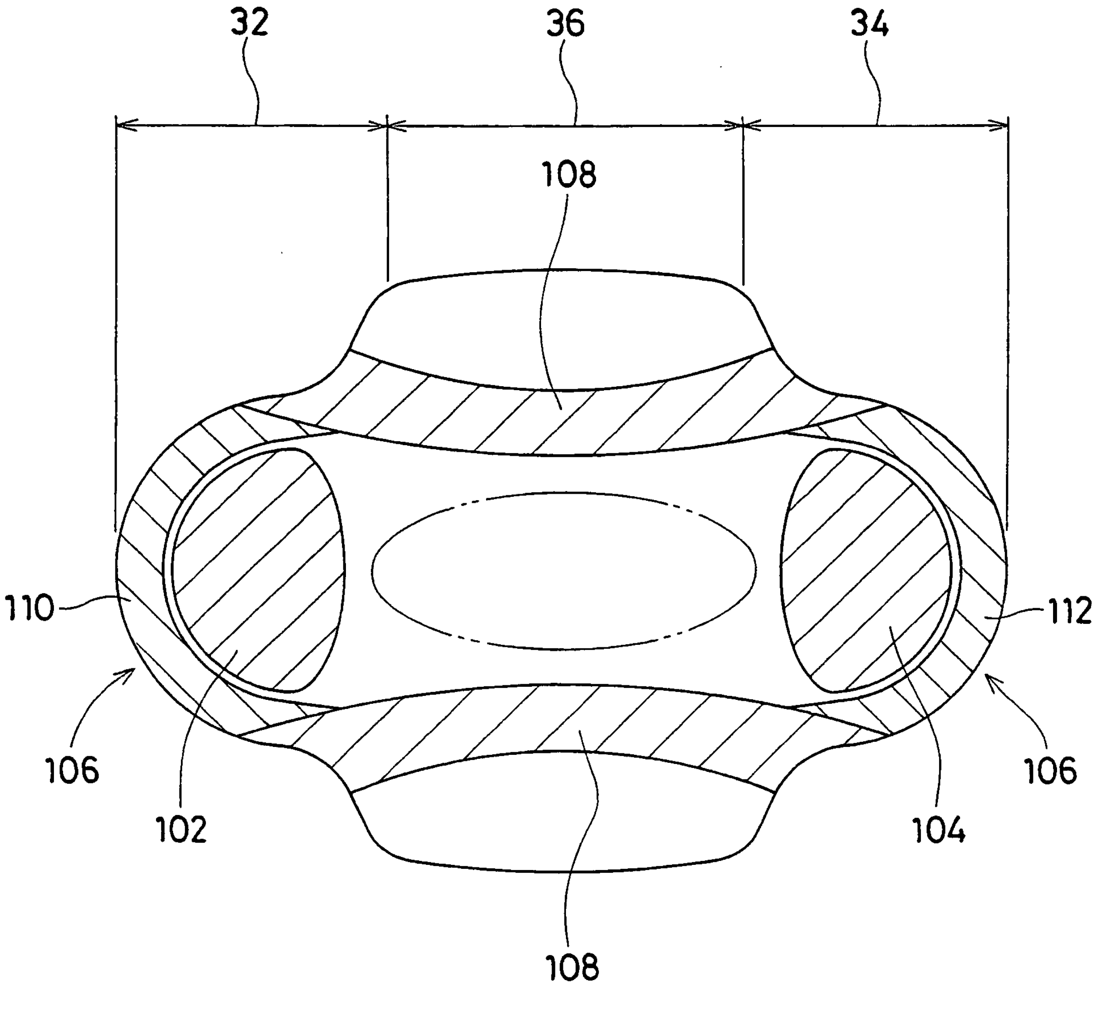


FIG. 5

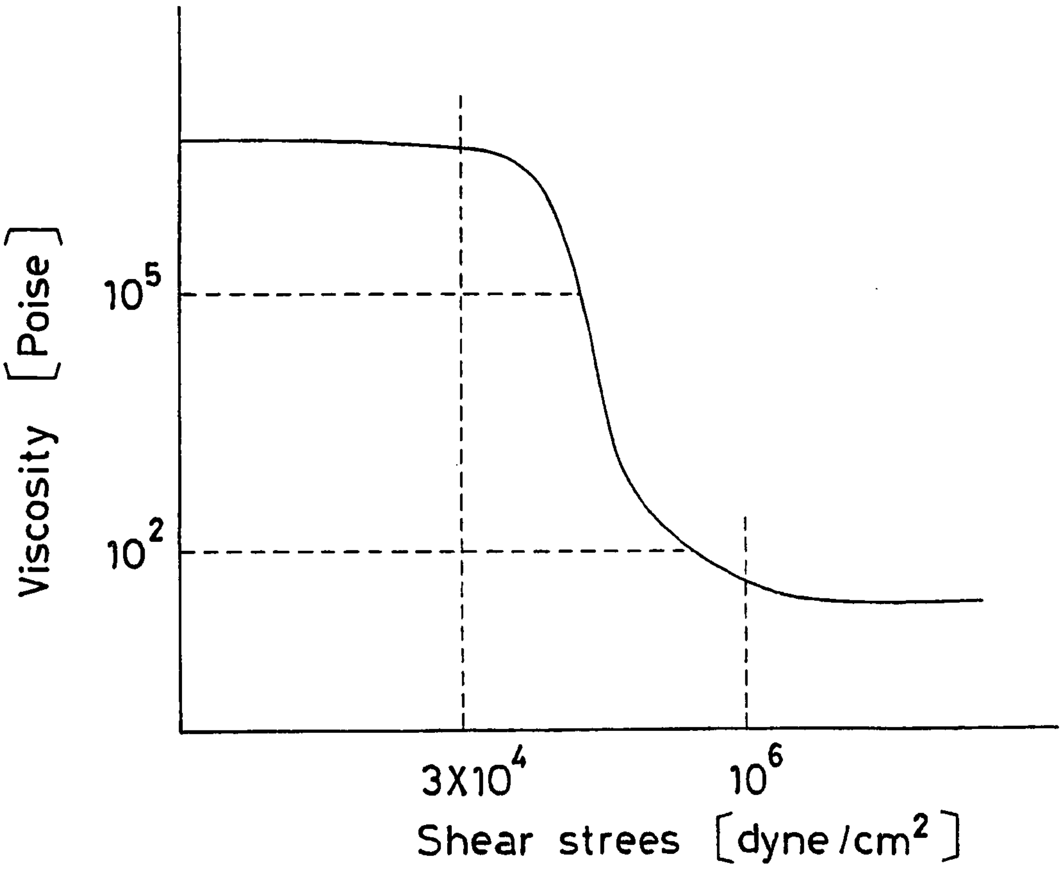


FIG. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4412833 A
- US 4413986 A
- US 2092346 A
- US 3905372 A
- US 2662527 A
- US 4631062 A
- US 2917049 A, Delaney
- US 3420235 A, Harmon
- US 4595392 A, Johnson
- US 5484429 A, Vukos
- US 3983873 A
- US 4175561 A, Hirschman
- US 4573986 A, Minetola
- US 3911173 A, Sprague, Jr.
- US 4785996 A, Zwieker
- US 4842666 A, Werenicz
- US 4690680 A, Higgins
- US 4950264 A
- US 5009653 A, Osborn
- US 3929135 A
- US 4324246 A
- US 4342314 A
- US 4463045 A
- US 4780352 A
- US 5006394 A
- US 44293595 A
- WO 9600548 A
- US 4950254 A, Osborn
- US 4988344 A
- US 4988345 A
- US 4777073 A, Exxon's
- US 5460623 A
- US 12211493 A
- WO 9507674 A
- US 5234422 A, Sneller
- US 4589876 B1
- US 4687478 A
- US 5389094 A
- US 5558663 A
- US 9615957 W
- US 4917697 A
- US 4946527 A
- US 5392498 A
- US 4556146 A, Swanson
- WO 9712576 A
- US 4425130 A
- US 1926900 A, Haas
- US 1946911 A, Haas
- US 3322123 A, Giswold
- US 5762644 A
- WO 9829078 A
- US 404814 A
- US 07142598 A, Brown
- US 4738676 A
- US 4253461 A, Strickland
- US 4597760 A
- US 4597761 A
- US 4704115 A
- US 4909802 A, Ahr
- US 4964860 A, Gipson
- US 63709091 A, Noel
- WO 9211830 A
- US 5643588 A
- US 5635191 A
- US 5609587 A
- US 5607760 A
- US 92653297 A
- US 92653397 A
- US 90885297 A
- US 76638696 A
- US 84003997 A
- US 96231097 A
- US 96231297 A
- US 86989797 A
- US 86970097 A
- US 92656697 A
- US 5059282 A, Ampulski
- US 5174927 A, Honsa
- US 04150998 A
- US 04126698 A
- US 5091193 A, Enjolras
- US 5376655 A, Imaki
- US 04119698 A
- US 041196 A
- US 3935862 A, Kraskin
- US 5409903 A, Polak
- US 4556560 A
- EP 97120699 A
- US 5643874 A
- EP 97120700 A
- US 04123298 A

**Non-patent literature cited in the description**


- **C. Michael White et al.** Cholestyramine Ointment to Treat Buttocks Rash and Anal Excoriation in an Infant. *The Annals of Pharmacotherapy,* September 1996, vol. 30, 954-956